(19) Europäisches Patentamt
European Patent Office
Office européen des brevets

(11) Publication number: **0 423 445 B1**

(12) # EUROPEAN PATENT SPECIFICATION

(45) Date of publication of patent specification: **08.11.95**

(51) Int. Cl.[6]: **C07D 493/22**, A61K 31/365, A01N 43/90, //(C07D493/22, 313:00,311:00,311:00,307:00), (C07D493/22,313:00,311:00, 311:00,307:00,303:00)

(21) Application number: **90114920.3**

(22) Date of filing: **03.08.90**

The file contains technical information submitted after the application was filed and not included in this specification

(54) **13-alkyl-23-imino and 13-halo-23-imino derivatives of LL-F28249 compounds and their use as endo- and ectoparasiticidal insecticidal, acaricidal and nematocidal agents.**

(30) Priority: **11.09.89 US 405808**
**22.12.89 US 455686**

(43) Date of publication of application:
**24.04.91 Bulletin 91/17**

(45) Publication of the grant of the patent:
**08.11.95 Bulletin 95/45**

(84) Designated Contracting States:
**AT BE CH DE DK ES FR GB GR IT LI LU NL SE**

(56) References cited:
**EP-A- 0 253 378**
**EP-A- 0 259 779**
**EP-A- 0 293 549**

**PATENT ABSTRACTS OF JAPAN, vol. 13, no. 255 (C606) (3603), 13th June 1989**

(73) Proprietor: **AMERICAN CYANAMID COMPANY**
**One Cyanamid Plaza**
**Wayne, NJ 07470-8426 (US)**

(72) Inventor: **Buckwalter, Brian Lee**
**102 Ovington Road**
**Yardley,**
**Pennsylvania (US)**
Inventor: **Tseng, Shin-Shyong**
**232 Windmill Court**
**Bridgewater,**
**New Jersey (US)**
Inventor: **Barden, Timothy Claude**
**3424 Stafford Place**
**Holland,**
**Pennsylvania (US)**

(74) Representative: **Wächtershäuser, Günter, Prof. Dr.**
**Patentanwalt,**
**Tal 29**
**D-80331 München (DE)**

**EP 0 423 445 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to certain 13-alkyl-23-imino and 13-halo-23-imino-LL-F28249 compounds and their use for controlling endo- and ectoparasitic infections and infestations in warm-blooded animals and controlling the housefly. The compounds may also be applied to a wide variety of agronomic crops and the surroundings in which the crops are grown or growing to protect the crops from the damage caused by insects, acarids and nematodes.

The designation LL-F28249 is used to describe compounds produced by the fermentation broth of Streptomyces cyaneogriseus, subspecies noncyanogenus, deposited in the NRRL collection under deposit accession number 15773. The morphological characteristics of said compounds and methods for the production thereof are described in co-pending patent application Serial Number 732,252 filed on May 10, 1985.

The compounds of the invention have the structural formula:

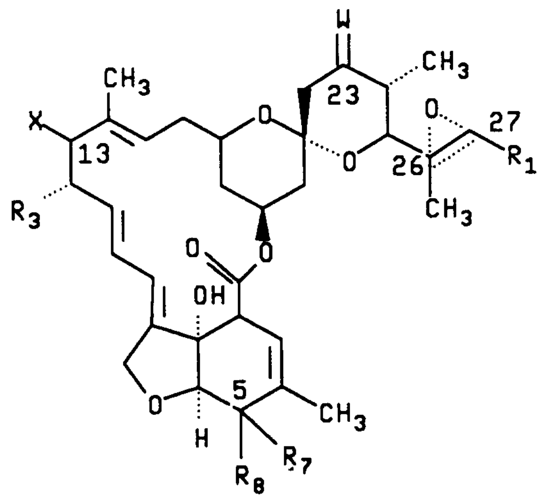

wherein

- $R_1$ is methyl, ethyl or isopropyl;
- $R_7$ is hydrogen and $R_8$ is $OR_2$, or when taken together with the carbon atom to which they are attached $R_7$ and $R_8$ represent C=0;
- $R_2$ is hydrogen, $C_1$-$C_4$ alkyl, or

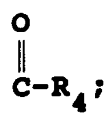

- $R_4$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy;
- $R_3$ is hydrogen, methyl or ethyl;
- X is fluorine, bromine, chlorine or iodine;
- W is N=Y;
- Y is $OR_5$ or $NHR_6$;
- $R_5$ is $C_1$-$C_4$ alkyl or $C_1$-$C_4$ acyl; and
- $R_6$ is $C_1$-$C_4$ acyl; and

the dotted triangular figure with oxygen at $C_{26}$-$C_{27}$ indicates that there is present either a double bond or an epoxide.

A preferred group of 13-halo-23-imino-LL-F28249 compounds have the structural formula shown above wherein

- $R_1$ is isopropyl;

$R_7$ is hydrogen and $R_8$ is $OR_2$;
$R_2$ is hydrogen, $C_1$-$C_4$ alkyl, or

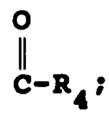

$R_4$ is hydrogen, methyl, chloromethyl, dichloromethyl, trichloromethyl, or methoxymethyl;
$R_3$ is methyl;
X is fluorine;
W is N=Y;
Y is $OR_5$; and
$R_5$ is $C_1$-$C_4$ alkyl.

In addition,the compounds of the invention have the structural formula:

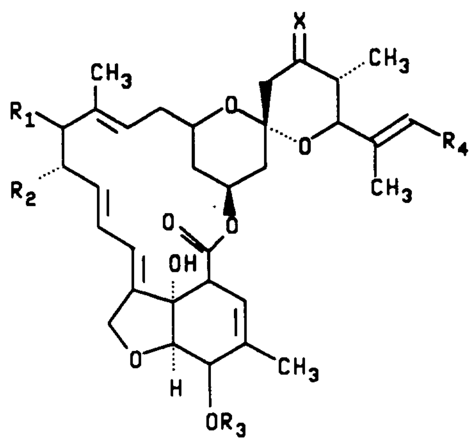

(I)

wherein
$R_1$ is $C_1$-$C_4$ alkyl;
$R_2$ is hydrogen, methyl or ethyl;
$R_3$ is hydrogen, $C_1$-$C_4$ alkyl or

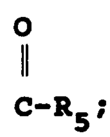

$R_5$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy groups;
$R_4$ is methyl, ethyl or isopropyl;
$R_6$ is hydrogen, $C_1$-$C_4$ alkyl, $C_1$-$C_4$ alkoxymethyl, $C_1$-$C_4$ alkanoyl, benzyl or phenyl;
X is oxygen, $NOR_6$ or $NNHR_7$;
$R_7$ is $C_1$-$C_4$ acyl, $C_1$-$C_4$ alkyl or benzoyl.

A preferred group of 13-alkyl-23-imino-LL-F28249 compounds have the structural formula shown above wherein
$R_1$ is methyl, ethyl or isopropyl;
$R_2$ is methyl;
$R_3$ is hydrogen, $C_1$-$C_4$ alkyl, or

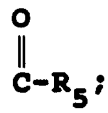

| | |
|---|---|
| $R_5$ | is hydrogen, $C_1$-$C_4$ alkoxymethyl, $C_1$-$C_4$ halomethyl or formyl; |
| $R_4$ | is isopropyl; |
| X | is $NOR_6$; and |
| $R_6$ | is $C_1$-$C_4$ alkyl. |

The LL-F28249 compounds are represented by the following structural formula:

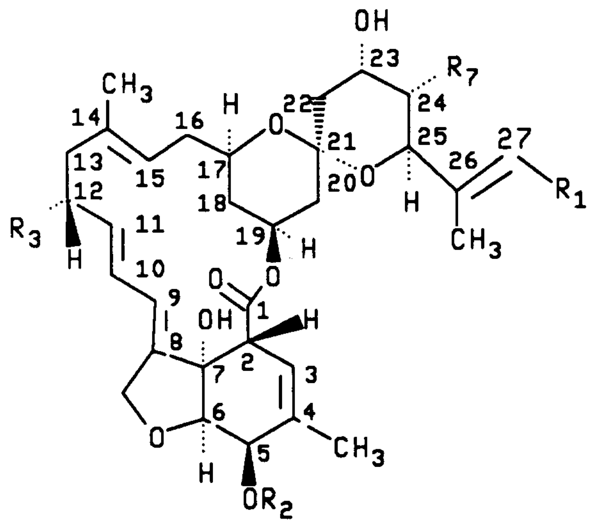

| Component | $R_1$ | $R_2$ | $R_3$ | $R_7$ |
|---|---|---|---|---|
| LL-F28249α | $CH(CH_3)_2$ | H | $CH_3$ | $CH_3$ |
| LL-F28249β | $CH_3$ | H | $CH_3$ | $CH_3$ |
| LL-F28249γ | $CH_3$ | $CH_3$ | $CH_3$ | $CH_3$ |
| LL-F28249ε | $CH(CH_3)_2$ | H | H | $CH_3$ |
| LL-F28249ζ | $CH_2CH_3$ | H | $CH_3$ | $CH_3$ |
| LL-F28249θ | $CH(CH_3)_2$ | H | $CH_3$ | $CH_2CH_3$ |
| LL-F28249ℓ | $CH(CH_3)_2$ | H | $CH_2CH_3$ | $CH_3$ |
| LL-F28249λ | $CH(CH_3)_2$ | $CH_3$ | $CH_3$ | $CH_3$ |

The compounds of the invention are effective antiendoparasitic and antiectoparasitic agents and can be used to protect warm-blooded animals against infections and infestations of the above-said pests. They may also be applied to a wide variety of agronomic crops and the surroundings in which the crops are grown or growing to protect the crops from the damage caused by insects, acarids and nematodes. They are highly effective for the control of the housefly when applied to the habitat, food source, or breeding ground of the housefly.

Surprisingly, it has been found that chemical modification at the 5, 13, 23, 26 and 27 positions of the LL-F28249 compounds shown above enhances the endo-and ectoparisiticidal, insecticidal, acaricidal and nematocidal activity of said compounds. More particularly, the 13-halo-23-imino derivatives of 5-hydroxy and 5-O-substituted-LL-F28249 and 26,27-epoxy-LL-F28249 compounds and the 13-alkyl-23-imino derivatives of 5-hydroxy and 5-0-substituted-LL-F28249 compounds demonstrate potent endectocidal and insecticidal activity.

Functionalization at the 13 position of the above shown 13-halo-23-imino derivatives of LL-F28249 compounds is achieved by oxidation of the 5 and 23 hydroxy groups with pyridinium dichromate in dimethylformamide to give the 5,23-dioxo-LL-F28249 compound (I) followed by reaction with selenium

dioxide and formic acid to give the 13-(formyloxy)-5,23-dioxo-F28249 compound (II). The thus-obtained compound (II) is converted to the 13-hydroxy-5,23-dioxo-LL-F28249 compound (III) via acid hydrolysis and then reacted with diamino sulfur trifluoride (DAST) to give the 13-fluoro-5,23-dioxo-LL-F28249 compound (IV). Compound (IV) is reacted sequentially with methoxylamine hydrochloride and sodium borohydride to give the 13-fluoro-23-methoximino-LL-F28249 compound (VI). Using LL-F28249α as the starting material, the above-described reaction sequence is illustrated in Flow Diagram I shown below.

FLOW DIAGRAM I

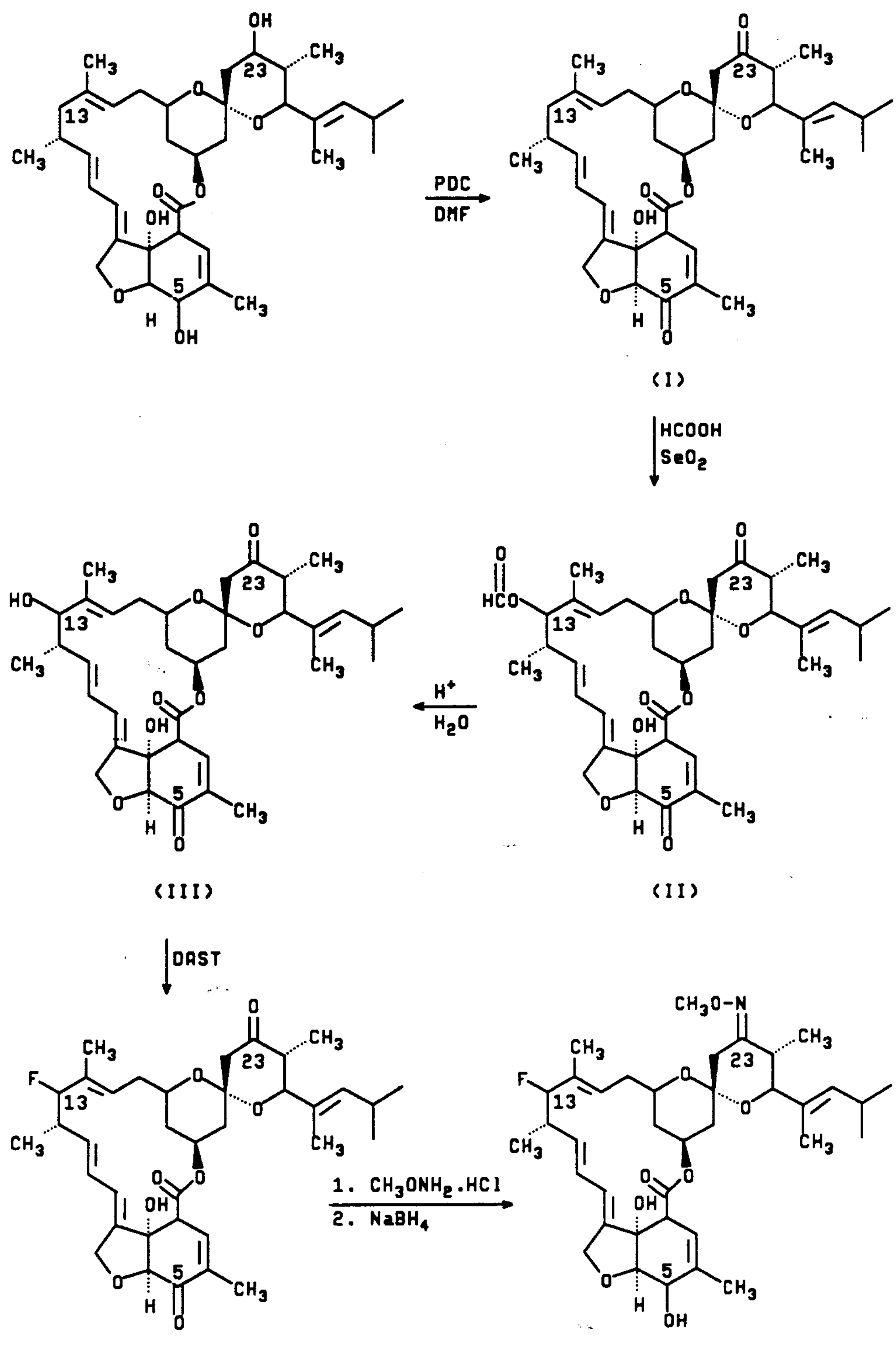


(I) (II) (III) (IV) (V)

Compound (III) is used as a common intermediate and reacted with halogenating reagents such as phosphorous tribromide and thionyl chloride to give the 13-bromo- or 13-chloro- or 13-iodo-5,23-dioxo-LL-F28249 compounds represented by compound (IV) wherein F is Br or Cl or I, and then reacted sequentially with an alkoxyamine, or hydroxylamine, and sodium borohydride to give the corresponding 13-halo-23-imino-LL-F28249 compounds represented by compound (V) wherein F is Br or Cl or I as shown in Flow

Diagram I, above.

Derivatization of the 5-hydroxy moiety to give novel 5-alkyloxy and 5-acyloxy-13-halo-23-imino-LL-F28249 compounds is achieved by reacting compound (VI) with an alkylating or acylating agent as illustrated in Flow Diagram II.

## FLOW DIAGRAM II

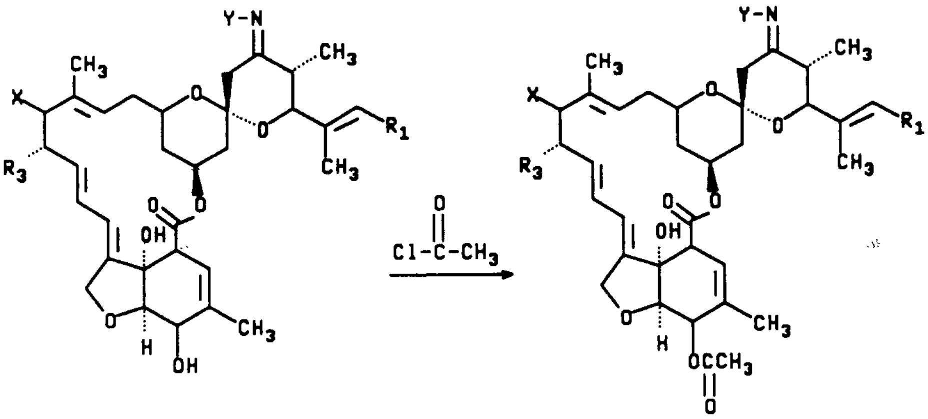


(VI)

$R_1$, $R_3$, X and Y are as described hereinabove.

Alternatively, compounds of the invention are prepared by reacting the LL-F28249 starting material with an acyl halide such as p-nitrobenzoylchloride followed by oxidation of the 23-hydroxy group and functionalization at the 13 carbon to give the intermediate compound (VII) as illustrated in Flow Diagram III.

## FLOW DIAGRAM III

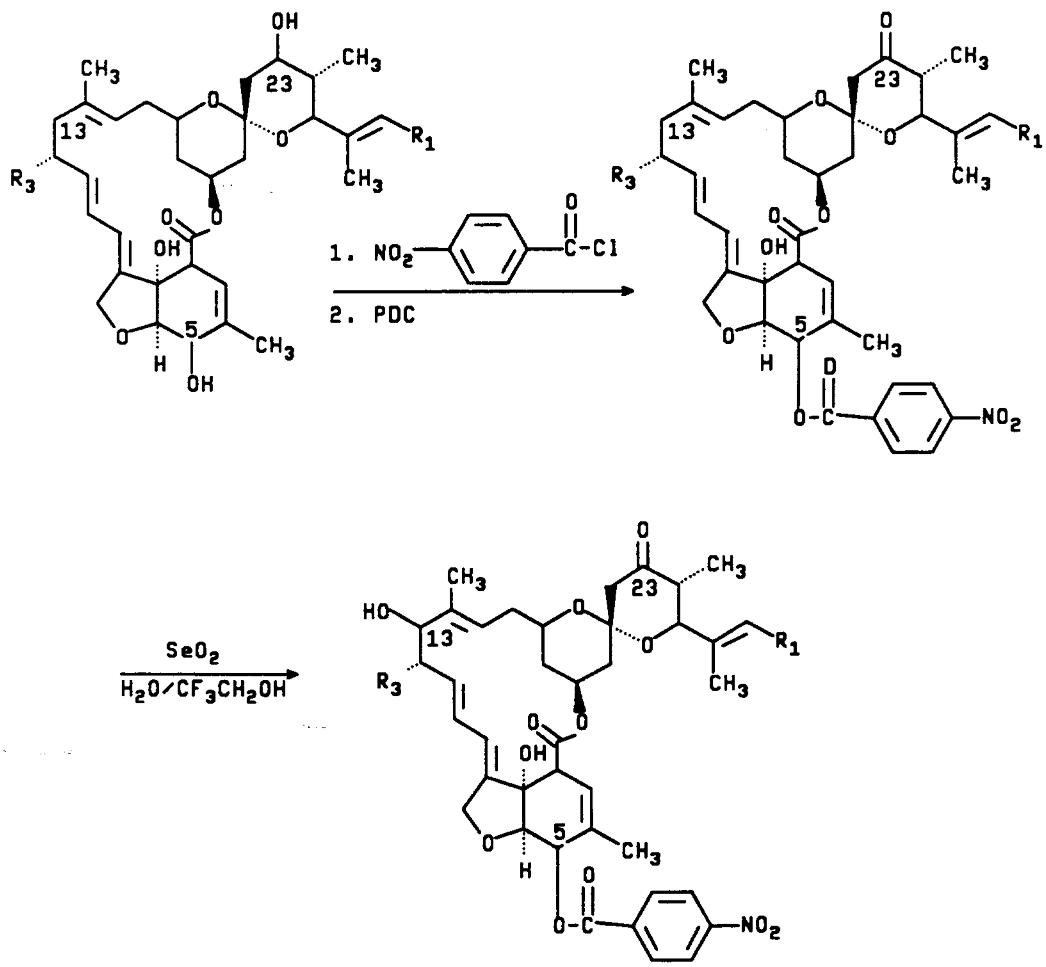


(VII)

Compound (VII) is treated with an halo-genating agent such as diamino sulfur trifluoride, thionyl chloride or phosphorous tribromide followed by reaction with an alkoxyl or hydroxylamine to give the desired 13-halo-23-imino-5-(acyloxy)-LL-F28249 compounds such as compound (VIII). Optionally, compound (VIII) is hydrolyzed in base to give the corresponding 13-halo-23-imino-LL-F28249 compounds. The reaction sequence is illustrated in Flow Diagram IV.

FLOW DIAGRAM IV

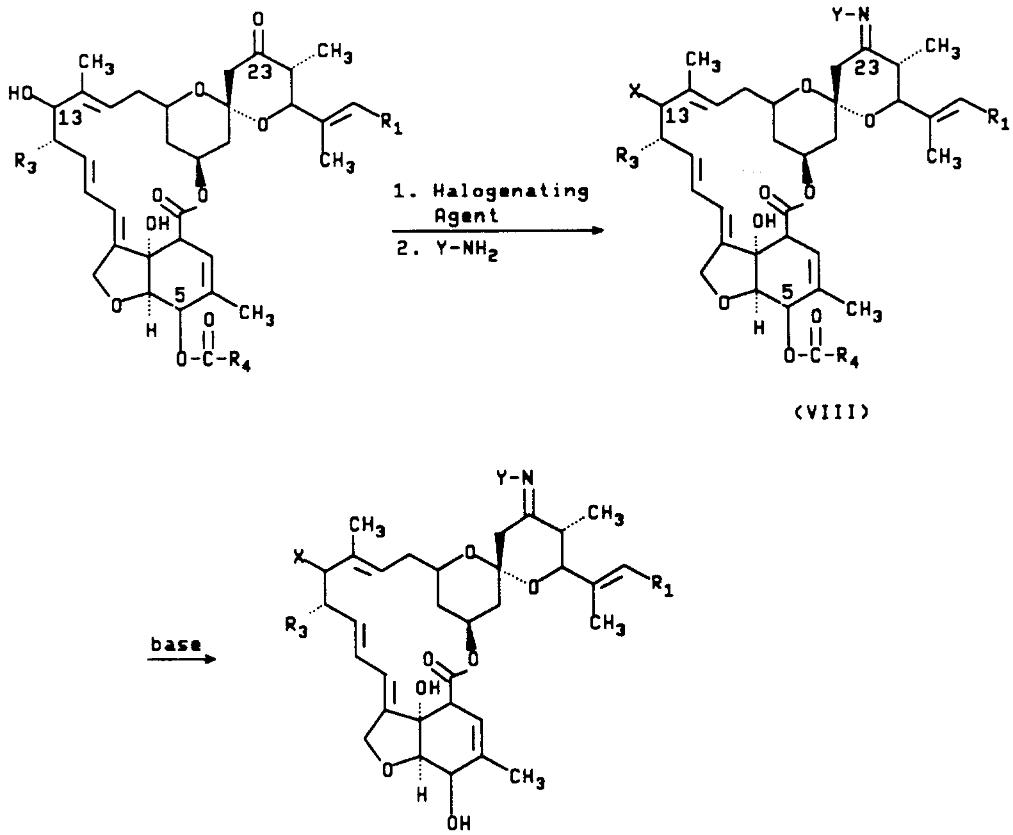


(VIII)

$R_1$, $R_3$, $R_4$, X and Y are as described hereinabove.

Advantageously, the 26,27 epoxide derivatives of 13-halo-23-imino-LL-F28249 compounds are prepared by reacting the 13-halo-23-oxo precursor with m-chloro-perbenzoic acid followed by treatment of the 26,27 epoxide product with an alkoxy, acyloxyl or hydroxyloxime to give the desired compounds (IX) as illustrated in Flow Diagram V.

FLOW DIAGRAM V

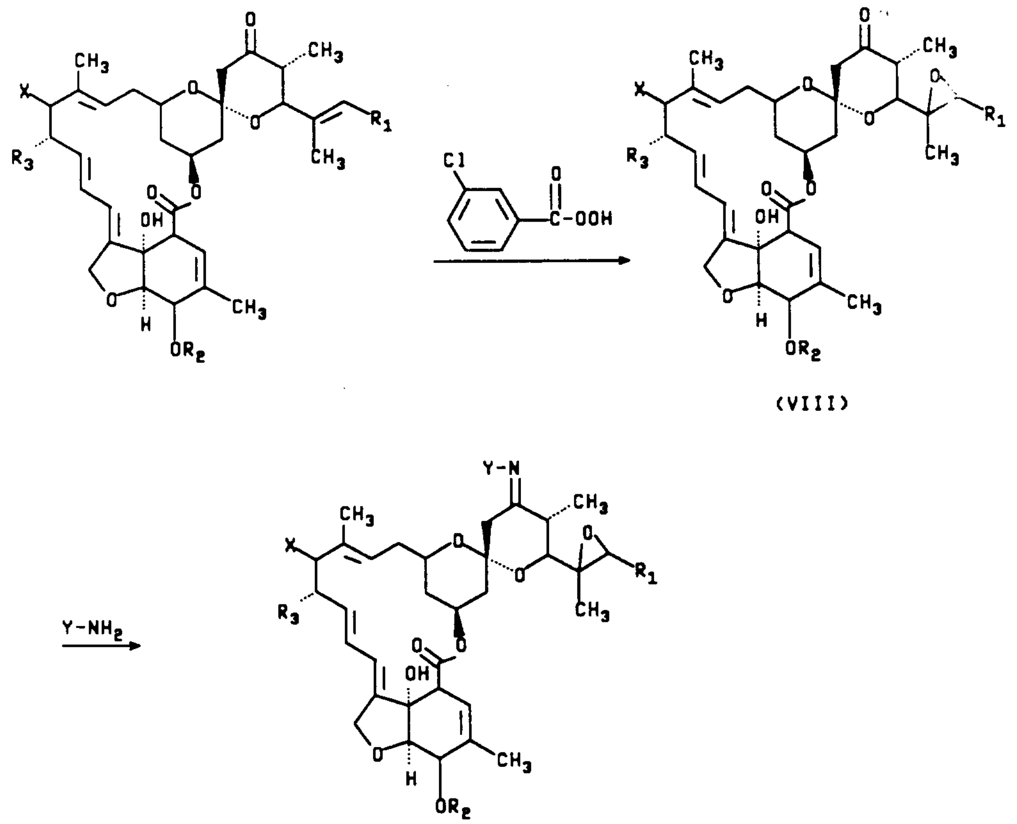


(VIII)

(IX)

$R_1$, $R_2$, $R_3$, X and Y are as described hereinabove.

The 13-alkyl-23-imino derivatives of LL-F28249 compounds of the present invention may be prepared by reacting LL-F28249 compounds with acetic anhydride to yield the 5-acetoxy-LL-F28249 compound (II). Reacting the 5-acetoxy-LL-F28249 compound with calcium carbonate and methyl oxalyl chloride followed by treatment with hydrochloric acid yields the 5-acetoxy-23-{[(methoxycarbonyl)-carbonyl]oxy}-LL-F28249 compound (III). The thus-obtained compound (III) is converted to the 5-acetoxy-13$\beta$-hydroxy-23-{[-(methoxycarbonyl) carbonyl]oxy}-LL-F28249 (IV) by treatment with formic acid and selenium dioxide followed by reaction with hydrochloric acid. Reacting the formula (IV) compound with ethyl chloroformate, pyridine and 4-dimethylaminopyridine yields the 5-acetoxy-13$\beta$-[(ethoxycarbonyl)oxy]-23-{[-(methoxycarbonyl)carbonyl]oxy}-LL-F28249 compound (V). Reacting the said formula (V) compound with trialkylaluminum yields a 5-acetoxy-13$\beta$-alkyl-LL-F28249 compound (VI). The formula (VI) compound is converted to a 5-acetoxy-13$\beta$-alkyl-23-oxo-LL-F28249 compound (VII) by treatment with 4-methylmorpholine N-oxide and tetrapropylammonium perruthenate and then reacted with an alxoxylamine hydrochloride to yield a 5-acetoxy-13$\beta$-alkyl-23-(O-alkyloxime)-LL-F28249 compound (VIII). Reacting compound (VIII) with a strong base, such as sodium hydroxide, yields a desired formula (I) 13$\beta$-alkyl-23-(O-alkyloxime)-LL-F28249 compound. This reaction scheme is illustrated in flow diagram VI:

**Flow Diagram VI**

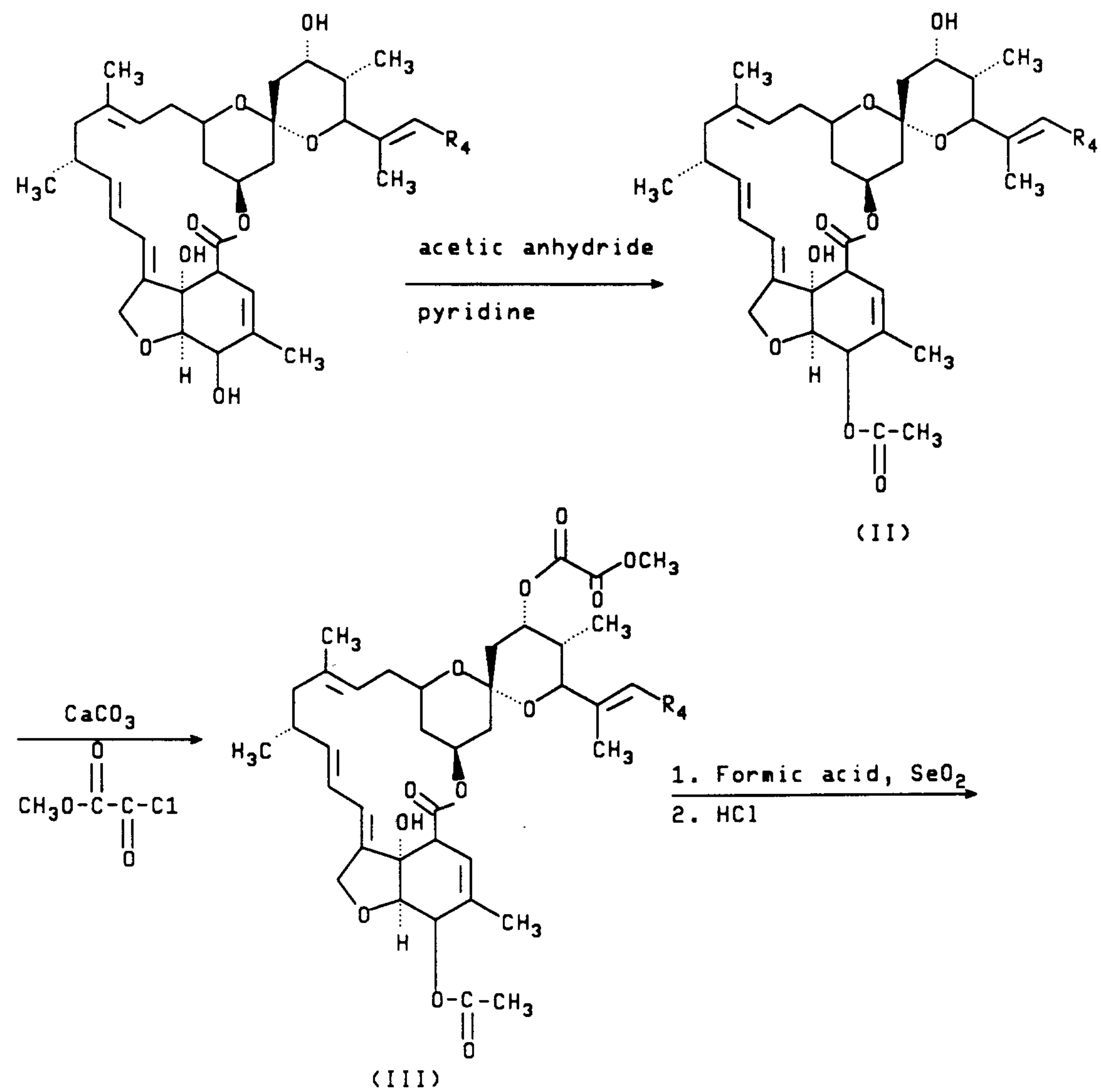


(II)

(III)

## Flow Diagram VI (Continued)

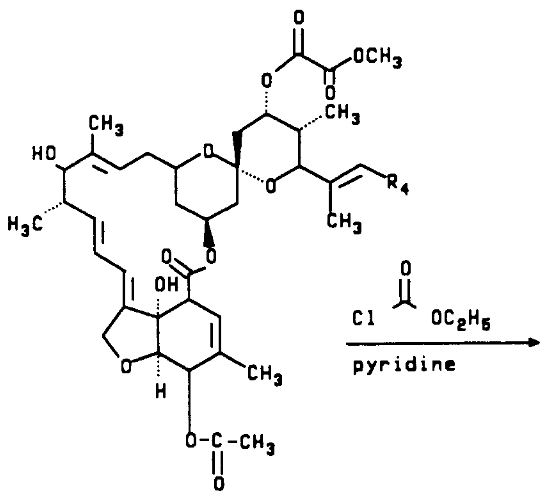


(IV)

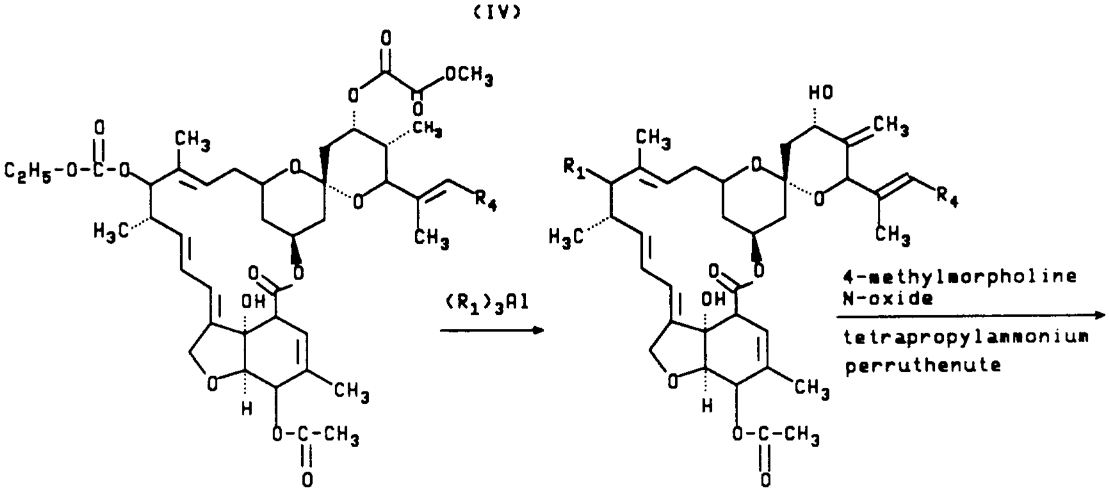


(V) (VI)

## Flow Diagram VI (Continued)

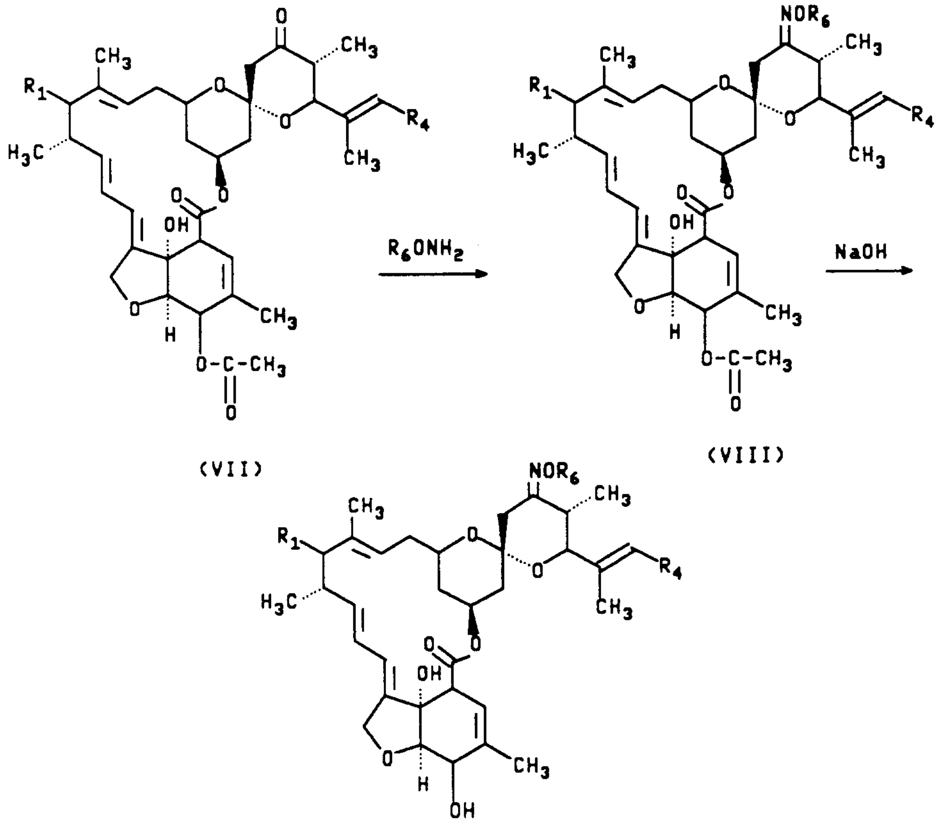


(VII)

(VIII)

(I)

Surprisingly, it has been found that the compounds of the invention are highly effective endo-and ectoparasiticidal agents useful for treating warm-blooded animals. It has also been found that said compounds are excellent insecticidal, nematicidal and acaricidal agents useful for controlling said pests and protecting agronomic crops from damage caused by said pests.

For use in the treatment of warm-blooded animals such as cattle, sheep, swine, horses, dogs or other mammals, the compounds of the invention may be administered orally in a dosage unit form such as a capsule, bolus or tablet, or as a liquid drench. The drench is normally a solution, suspension or dispersion of the active ingredient usually in water together with a suspending agent such as bentonite and a wetting agent or like excipient and an antifoaming agent. Drench formulations generally contain from 0.001 to 0.5% by weight of the active compound. The capsules and boluses comprise the active ingredient admixed with a carrier vehicle such as starch, talc, magnesium stearate or di-calcium phosphate.

Where it is desired to administer the LL-F28249 derivatives in a dry, solid dosage unit form, capsules, boluses or tablets containing the desired amount of active compound usually are employed. These dosage

forms are prepared by intimately and uniformly mixing the active ingredient with suitable finely divided diluents, fillers, disintegrating agents and/or binders such as starch, lactose, talc, magnesium stearate, vegetable gums and the like. Such dosage unit formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparasitic compounds of the invention may be administered to animals parenterally, for example, by intraruminal, intramuscular, intratracheal, or by subcutaneous injection, in which event the active ingredient is dissolved or dispersed in a liquid carrier vehicle. For parenteral administration, the active material is admixed with an acceptable vehicle, preferably a vegetable oil such as peanut oil, cotton seed oil or the like. Other parenteral vehicles such as organic preparations using solketal, propylene glycol, glycerol formal and aqueous parenteral compositions may also be used in the preparation of parenteral formulations for administration to warm-blooded animals.

In these formulations the active compound or compounds are dissolved or suspended in the formulation in sufficient amount to provide from 0.005% to 5% by weight of the active compound in said formulation.

The compounds of the invention are useful for treating endoparasites in warm-blooded animals such as helminths. Said compounds are also useful for the prevention and treatment of diseases caused by ectoparasites, for example, arthopod ectoparasites such as mites, ticks, lice, fleas and other biting insects in domesticated animals and poultry.

Further, the 13-halo-23-imino-LL-F28249 compounds described herein are excellent insecticidal, nematicidal and acaricidal agents useful for protecting, growing or harvested crops from attack by the above-said pests. The compounds of the invention may be formulated into dry compacted granules, flowable compositions, wettable powders, dusts, dust concentrates, microemulsions and the like, all of which lend themselves to soil, water and/or foliage application and provide the requisite plant protection. Such compositions include the compounds of the invention admixed with agronomically acceptable solid or liquid carriers.

It has also been found that the compounds of the invention exhibit surprising insecticidal, nematicidal and acaricidal properties, making them useful for controlling insects, nematodes and acarids and protecting agronomic crops, trees, shrubs, stored grain and ornamental plants from damage caused by insects, nematodes and acarids.

For plant application, the compounds of the invention may be formulated into dry compacted granules, flowable compositions, wettable powders, dusts, dust concentrates, microemulsions and the like, all of which lend themselves to soil, water and/or foliage application and provide the requisite plant protection. Such compositions include the compounds of the invention admixed with agronomically acceptable solid or liquid carriers.

In these compositions the compounds are intimately mixed or ground together with the composition in sufficient amounts to provide from 3 to 20% by weight of the active compound in said composition.

The compositions of this invention are useful in combating agricutlrual pests that inflict damage upon crops while they are growing or while in storage. The compounds are applied using known technicques such as sprays, dusts, emulsions, wettable powders, flowables and the like to the growing or stored crops to provide protection against infestation by agricultural pests.

Still further, compounds of this invention are outstandingly effective in the control of the housefly in the habitat, food source or breeding ground of said housefly. The compounds can be applied using sprays, dusts, emulsions, wettable powders, flowables, microemulsion granular compositions and the like as described hereinabove to said habitat, food source or breeding ground of said housefly.

In order to facilitate a further understanding of the ivention, the following examples are presented primarily for the purpose of illustrating certain more specific details thereof. The invention is not to be limited thereby except as defined in the claims.

Unless otherwise noted, all parts are by weight.

## EXAMPLE 1

### Preparation of 5,23-dioxo-LL-F28249α

A mixture of LL-F28249α (10.0g, 0.016 mole) and diatomaceous earth (160.0 g) in dimethylformamide, under nitrogen, is treated portionwise with pyridinium dichromate (58.0g, 0.15 mole), stirred for 4 hours at room temperature, treated with 1L of diethyl ether, stirred for 15 minutes and filtered. The filtrate is washed

with water followed by brine, dried over $MgSO_4$ and concentrated in vacuo to give a yellow oil residue. The residue is flash chromatographed (silica, methylene chloride:isopropanol 99:1 as eluent) to afford the title product as a light yellow solid (4.84 g, 49.7%), identified by $^1$HNMR, $^{13}$CNMR and mass spectral analyses.

**EXAMPLE 2**

**Preparation of 13-(formyloxy)-5,23-dioxo-LL-F28249α**

A mixture of 5,23-dioxo-LL-F28249α (2.6 g, 4.3 mmole) in 88% formic acid, under nitrogen, is treated with selenium dioxide (1.0 g, 9.0 mmole), stirred at 50° for 2 hours, cooled to room temperature, treated with 3 volumes of methylene chloride, stirred for 10 minutes and filtered through diatomaceous earth. The filtrate is separated and the organic phase is concentrated in vacuo to give a light brown solid residue. Flash column chromatography (silica, hexanes:ethyl acetate, 2:1 as eluent) of the residue affords the title product as a beige solid (1.3 g, 43%), identified by $^1$HNMR and $^{13}$CNMR spectroscopy.

**EXAMPLE 3**

**Preparation of 13-hydroxy-5,23-dioxo-LL-F28249α**

A mixture of 13-(formyloxy)-5,23-dioxo-LL-F28249α (800 mg, 1.1 mmole), methanol and dioxane, at 0-5°C is treated with 20 mL of 1.2N HCl, stirred at room temperature for 4 hours, allowed to stand at 40°C for 16 hours and concentrated in vacuo. The residue is taken up in ethyl acetate, washed with sodium bicarbonate solution followed by brine, dried over $MgSO_4$ and concentrated in vacuo, to give the title compound as a beige solid (700 mg, 91%), identified by $^1$HNMR, $^{13}$CNMR and mass spectral analyses.

**EXAMPLE 4**

**Preparation of 13-fluoro-5,23-dioxo-LL-F28249α and 13-fluoro-23-oxo-LL-F28249α**

A mixture of 13-hydroxy-5,23-dioxo-LL-F28249α (100 mg, 0.15 mmole) in methylene chloride at -40°C, under nitrogen, is treated with dimethylaminosulfur trifluoride (40 µl, 29.4 mg, 0.22 mmole) via a syringe and stirred at -40°C to -30°C for 3 hours. The reaction mixture is poured into ice water, and the phases are separated. The aqueous phase is extracted with ethyl acetate. The organic phases are combined, washed with water and brine, dried over $MgSO_4$ and concentrated in vacuo to give the title product mixture as a yellow solid, 112 mg. High pressure liquid chromatography (HPLC) analysis indicates 57% 13-fluoro-5,23-dioxo-LL-F28249α and 13% 13-fluoro-23-oxo-LL-F28249α is present in the mixture. Column chromatography (silica, hexanes:ethyl acetate, 4:1 as eluent) of the product mixture affords pure 13-fluoro-5,23-dioxo-LL-F28249α as a yellow solid, identified by $^1$H, $^{13}$C and $^{19}$F NMR and mass spectral analyses.

**EXAMPLE 5**

**Preparation of 13-fluoro-23-(O-methyloxime)-LL-F28249α**

A mixture of 13-fluoro-5,23-dioxo-LL-F28249α (30 mg, 0.05 mmole) in absolute ethanol at -10°C to -5°C, under nitrogen, is treated with imidazole (10 mg, 0.15 mmole) followed by addition of methoxylamine hydrochloride (12.5 mg, 0.15 mmole), stirred for 2 hours at -10°C to -5°C, treated with sodium borohydride (12.0 mg, 0.30 mmole) and stirred for 1 hour at -10°C to -5°C. The reaction mixture is quenched with water, stirred for 15 minutes at ambient temperatures and concentrated in vacuo. The resultant residue is dispersed in ethyl acetate and water, the phases are separated and the organic phase is washed with water followed by brine, dried over $MgSO_4$ and concentrated in vacuo to give a residue. The residue is column chromatographed (silica, hexanes: ether, 4:1 as eluent) to afford the title product as a white solid, identified by $^1$H, $^{13}$C and $^{19}$F NMR and mass spectral analysis.

**EXAMPLE 6**

**Preparation of 13-fluoro-5-(formyloxy)-23-(O-methyloxime)-LL-F28249α**

A mixture of 13-fluoro-23-(O-methyloxime)-LL-F28249α (80 mg, 0.12 mmole) in dry pyridine at 0°C, under nitrogen, is treated with 1.0 mL of the mixed anhydride of acetic acid and formic acid, stirred at room temperature for 1/2 hour then poured onto a mixture of ice and saturated sodium bicarbonate solution. The reaction mixture is extracted with ether, the ether extract is washed with cold dilute hydrochloric acid solution, followed by a water wash and a saturated sodium bicarbonate wash. The organic phase is dried over $Na_2SO_4$ and concentrated in vacuo. The resultant residue is flash chromatographed (silica, methylene chloride:ethyl acetate, 40:1 as eluent) to afford the title product as a white solid (34.4 mg, 42%) identified by $^1H$ and $^{13}C$ NMR and mass spectral analyses.

**EXAMPLE 7**

**Preparation of 5-(dichloroacetoxy)-13-fluoro-23-(O-methyloxime)-LL-F28249α**

A stirred mixture of 13-fluoro-23-(O-methyl-oxime)-LL-F28249α (85 mg, 0.12 mmole) and dimethylaminopyridine (2.9 mg, 0.024 mmole) in methylene chloride at 5-10°C, under nitrogen, is treated with pyridine (50lL, 0.62 mmole) followed by dichloroacetyl chloride (0.02 lL, 0.208 mmole). After 2 hours at 5-10°C, the reaction mixture is poured onto saturated sodium bicarbonate and ice and extracted with ether. The organic phase is washed sequentially with dilute (0.5%) hydrochloric acid, water and saturated sodium bicarbonate solution, dried over $Na_2SO_4$ and concentrated in vacuo. The residue is flash chromatographed (silica, 0.25% to 0.5% isopropanol in methylene chloride, gradient elution) to give the title product as a white solid, identified by $^1H$ and $^{13}C$ NMR and mass spectral analyses.

**EXAMPLE 8**

**Preparation of 13-fluoro-23-(O-methyloxime)-5-(trichloroacetoxy)-LL-F28249α**

A stirred mixture of 13-fluoro-23-(O-methyloxime)-LL-F28249α (80 mg, 0.12 mmole) and dimethylaminopyridine (2.9 mg, 0.024 mmole) in methylene chloride, at 5-10°C under nitrogen, is treated with pyridine (50 lL, 0.62 mmole) followed by trichloroacetyl chloride (20 lL, 0.18 mmole). After 1 1/4 hours at 5-10°C, the reaction mixture is poured into saturated sodium bicarbonate and ice and extracted with ether. The organic phase is washed sequentially with dilute (0.5%) hydrochloric acid, water and saturated sodium bicarbonate, dried over $Na_2SO_4$ and concentrated in vacuo to give a white foam residue. The residue is flash chromatographed (silica, 0.10% to 0.25% isopropanol in methylene chloride, gradient elution) to afford the title product as an off-white solid (63.4 mg, 66%) identified by $^1H$ and $^{13}C$ NMR and mass spectral analyses.

**EXAMPLE 9**

**Preparation of 5-[(p-nitrobenzoyl)oxy]-LL-F28249α**

A mixture of LL-F28249α (6.36 g, 10.4 mmole) and pyridine (1.98 g, 25 mmole) in methylene chloride at 20-25°C is treated with p-nitrobenzoyl chloride (2.45 g, 13.2 mmole), stirred for 4 hours, allowed to stand for 16 hours and treated with saturated sodium bicarbonate and methylene chloride. The reaction mixture is stirred for 5 minutes and the phases are separated. The organic phase is washed sequentially with saturated sodium bicarbonate, 5% hydrochloric acid and brine, dried over $MgSO_4$ and concentrated in vacuo to afford the title product as a white solid foam (7.9 g, 93.5% purity) by liquid chromatography analysis) identified by $^1H$NMR and mass spectral analyses and microanalysis.

**EXAMPLE 10**

**Preparation of 5-[(p-nitrobenzoyl)oxy]-23-oxo-LL-F28249α**

A stirred mixture of 5-[(p-nitrobenzoyl)oxy]-LL-F28249α (6.3 g, 8.4 mmole) in dimethylformamide is treated with pyridinium dichromate all at once at 20-25°C. The reaction mixture is stirred for 6 hours, poured

into water, stirred for 15 minutes and filtered. The filter cake is washed with water, air-dried, taken up in refluxing ethyl acetate, treated with diatomaceous earth and filtered. The filtrate is concentrated in vacuo to give a red-brown solid residue. The residue is recrystallised from n-propanol to afford the title product as white crystals (3.3 g, 51.7%) identified by $^{1}$HNMR and mass spectral analyses.

**EXAMPLE 11**

**Preparation of 13-hydroxy-5-[(p-nitrobenzoyl)oxy]-23-oxo-LL-F28249α**

A stirred mixture of 5-[(p-nitrobenzoyl)oxy]-23-oxo-LL-F28249α (1.25 g, 1.36 mmole) and selenium dioxide (0.78 g, 7.02 mmol) in 20 mL of 9:1 trifluoroethanol:water is heated at 40°C for 4 hours, cooled to room temperature and filtered through diatomaceous earth. The filtrate is concentrated in vacuo; the resultant residue is taken up in methylene-chloride and filtered to remove any residual selenium dioxide and the filtrate is concentrated in vacuo; the resultant residue is flash chromatographed (silica ethyl acetate:hexanes 1:2 as eluent) to afford the title product as a white solid (0.29 g, 27%) identified by $^{1}$H and $^{13}$CNMR and mass spectral analyses.

**EXAMPLE 12**

**Preparation of 13-fluoro-5-[(p-nitrobenzoyl)oxy]-23-oxo-LL-F28249α**

A mixture of 13-hydroxy-5-[(p-nitrobenzoyl)oxy]-23-oxo-LL-F28249α (0.5 mmole) in dry methylene chloride, under nitrogen, at -70°C is treated dripwise with dimethylamino sulfur trifluoride (80 ll, 0.60-mmole) via a syringe. After 20 minutes at -70°C, the reaction mixture is quenched with 1/2 mL of water and 1 mL of methanol and stirred for 15 minutes at -78°C to 0°C, allowed to-warm to room temperature and concentrated in vacuo. The residue is flash chromatographed (silica, methylene chloride/ethyl acetate mixtures as eluent) to afford the title product as a white solid (236 mg, 58.7%) identified by $^{1}$H, $^{13}$C and APT NMR and mass spectral analyses.

**EXAMPLE 13**

**Preparation of 13-fluoro-23-oxo-LL-F28249α**

A rapidly stirred mixture of 13-fluoro-5-[(p-nitrobenzoyl)oxy]-23-oxo-LL-F28249α (187 mg, 0.24 mmole) in dioxane at 5°C is treated dropwise with 1N NaOH (0.35 mL, 0.35 mmol), stirred at 10-15°C for 3 hours, diluted with ethyl acetate and water, and mixed well. The phases are separated and the organic phase is washed with water, dried over $NaSO_4$ and concentrated in vacuo. The residue is flash chromatographed (silica, ethyl acetate:hexane, 1:2-1:1 gradient elution) to give the title product as a white powder (117 mg, 78%), identified by $^{1}$H and $^{13}$CNMR and mass spectral analyses.

**EXAMPLE 14**

**Preparation of 26,27-epoxy-13-fluoro-23-oxo-LL-F28249α (I) and 3,4:26,27-diepoxy- 13-fluoro-23-oxo-LL-F28249α (II)**

A stirred mixture of 13-fluoro-23-oxo-L-L-F28249α (62.8 mg, 0.10 mmole) in methylene chloride at 5-10°C is treated with purified m-chloroperbenzoic acid (41.7 mg, 0.24 mmole), stirred for 3 hours at 5-10°C and quenched with 10% sodium bicarbonate solution. The phases are separated and the organic phase is concentrated in vacuo. The resultant residue is flash chromatographed (silica, hexanes:ethyl acetate gradient elution, 2:1-1:1) to give the title product mixture. The mixture is further purified by preparative, reverse phase, high pressure liquid chromatography (HPLC) (C-18 column, acetonitrile:water, 65:35) to afford title product (I) as a white powder (18 mg, 29%) and title product (II) as a white powder (16 mg, 26%). Both compounds (I) and (II) are identified by $^{1}$H and $^{13}$C NMR and mass spectral analyses.

**EXAMPLE 15**

**Preparation of 26,27-epoxy-13-fluoro-23-(O-methyloxime)-LL-F28249α**

A mixture of 26,27-epoxy-13-fluoro-23-oxo-LL-F28249α (7.6 mg, 0.012 mmole), hydroxylamine hydrochloride (4.5 mg, 0.05 mmole) and sodium acetate (6.6 mg, 0.08 mmole) in methanol is stirred at room temperature for 6 hours and concentrated in vacuo. The residue is partitioned between water and methylene chloride. The organic phase is separated and concentrated in vacuo. The resultant residue is chromatographed (silica, ethyl acetate:hexanes, 1:1) to afford the title product as a white powder (6.0 mg, 76%) identified by $^1H$ and $^{13}C$ NMR and mass spectral analyses.

**EXAMPLE 16**

**Preparation of 13-chloro-5,23-dioxo-LL-F28249α**

A mixture of 13-hydroxy-5,23-dioxo-LL-F28249α (70 mg, 0.10 mmole) in methylene chloride, under nitrogen, at 5-10°C is treated dripwise with thionyl chloride (30 lL, 18.4 mg, 0.15 mmole) via a syringe, stirred at 5-10°C for 2 hours and at room temperature for 16 hours and concentrated in vacuo. The semi-solid residue is flash chromatographed (silica, hexane:ethyl acetate, 75:25) to afford the title product as a beige solid (38.5 mg, 60%) identified by $^1H$NMR and mass spectral analyses.

**EXAMPLE 17**

**Preparation of 13-bromo-5,23-dioxo-LL-F28249α**

A mixture of 13-hydroxy-5,23-dioxo-LL-F28249α (97 mg, 0.15 mmole) in benzene, under nitrogen, at 5-10°C is treated with phosphorous tribromide (20 lL, 57.6 mg, 0.21 mmole) via a syringe, stirred for 1 hour at 5-10°C, poured into water and extracted with ethyl acetate. The organic phase is washed with brine, dried over $MgSO_4$, and concentrated in vacuo. The semi-solid residue is chromatographed twice (silica, hexanes:ethyl acetate, 9:1) to afford the title product as a light yellow solid identified by mass spectral analysis.

**EXAMPLE 18**

**Evaluation Of The Antiparasitic Activity Of Test Compounds.**

The antiparasitic activity of the compounds of the present invention against helminths, acarids, and arthropod ectoparasites at various concentrations of active ingredient is determined by the following test examples. The results of these tests are summarized in Table I.

**Evaluation of test compounds for controlling Trichostrongylus colubriformis in warm-blooded animals**

In these tests the active ingredient is dissolved in polyethylene glycol and dimethylsulfoxide (PEG:DMSO) (1:2 v/v) in sufficient quantity to provide treatments that deliver from 0.0156 to 0.1250 mg/kg of test compound to the animal.

To evaluate the test compounds, 5 week old male gerbils are infected with 400-600 infective T. colubriformis larvae of sheep origin on day 0. On day 7, the gerbils are weighed and treatment initiated. Test compounds are administered by gavage on the 7th day after treatment. The gerbils are sacrificed on the 11th day after treatment, and the remaining worms counted. The percent efficacy is calculated by comparing worm counts in treated animals with those from untreated infected controls using the following formula.

$$\frac{\text{Control mean} - \text{treated mean}}{\text{control mean}} \times 100 = \%\ \text{Efficacy}$$

Three replicates per treatment are employed in these evaluations.

The data obtained are summarized in Table I.

**Evaluation of test compounds for controlling Psoroptes cuniculi (Ear mites)**

On the day prior to test, or the morning of test, test compounds are dissolved in acetone and diluted to the desired concentrations. The concentration should be calculated so that 400 lL contains the amount to be placed on each filter paper. 400 lL of this solution is pipetted onto a top (3.7 cm dia) and bottom (3.5 cm dia) filter paper disc which is then placed on a ceramic plate to dry. [NOTE: This should be done under a hood.] There is a rough and smooth side to the filter paper. The test solution should be applied to the rough side which is placed up while drying. When dry, the two discs are placed in a Petri dish with the rough sides facing in separated by a small piece of stiff paper folded in the shape of a tent. Dishes are held at room temperature overnight, if done the day before the test. A standard at 0.01, 0.1 and 1.0 $lL/cm^2$ is run in each test.

Scab (containing mites) is collected from the ears of infested rabbits the morning of the test. This material is placed in a large Petri dish under an illuminated magnifier. Mites crawl out of the scab and are easily collected on the point of a dissecting needle or one prong of a pair of fine forceps. The top filter paper in each dish is removed and 12 mites are placed on the bottom disk and the top paper replaced. Before replacing the top of the Petri dish the rim of the dish is smeared with Vaseline to trap any escaping mites.

For evaluation tests there are generally 4 replicates of each dose which are counted, 2 at 4 hours and 2 at 24 hours.

After mites are added to the dishes, the dishes in each replicate are placed in a tray which is then placed in a plastic bag with several wet towels and held at room temperature.

After 4 or 24 hours, dishes are examined under a dissecting scope as follows -

1. Open dish carefully, remove top filter paper and save.
2. Draw a small circle approximately 1/2 cm in diameter on bottom filter paper using a soft pencil.
3. Using a disposable pipette, gently wet the area in and around the circle.
4. Transfer all mites from the dish into this circle. Look carefully - on cover, top filter paper and under bottom paper for mites.
5. Count and record the number of mites in the circle.
6. Replace the top cover and set the dish aside.
7. A minimum of 15 minutes later, count the mites remaining in the circle (these are dead mites).
8. Calculate and record the number of live mites.
9. Calculate percent efficacy as follows:

$$\frac{\text{Total of Dead mites}}{\text{Total number of mites}} \times 100 = \%\ \text{Efficacy}$$

The data obtained are summarized in Table I.

**Evaluation of test compounds for controlling Musca domestica**

In this test system, newly emerged housefly larvae are grown on an undefined medium of fermented whole milk and dried beef blood. Test compounds are added to the milk and activity is determined by the failure of larvae to pupate.

The test is run in 1 oz plastic medicine cups. Paper toweling (Scott C-Fold towels, #150) is cut in pieces approximately 3.5 x 10 cm. Three of these pieces are folded accordion style and placed in each paper cup. Twenty-four hours before the initiation of the test, 1/2 gallon of whole milk is divided into 5 1-liter beakers and placed in an incubator at 39°C. The morning of the test, the fermented milk is removed from the incubator, stirred and poured into small beakers (100 mL each). The l to 2 g of dried beef blood is added to each beaker with stirring to distribute the blood and incubated.

Doses of test compounds are calculated so that 0.1 mL contains the amount of test compound to be added to 100 mL of milk. Compounds should be dissolved in acetone. 100 lL of acetone is added to each control beaker.

Each beaker is removed from incubator and placed on a magnetic stirrer. The test compound is added and throughly mixed. Then 20 mL portions are removed and added to labelled test cups (4 reps/treatment). Using a fine paint brush, 20 larvae are transferred into each cup which is then sealed in a plastic bag with a few pinholes in it. Bags containing cups are placed on a flat tray and placed in a 27°C incubator for one week.

Trays are removed from incubator and the number of pupae in each bag recorded. The paper toweling is removed and examined closely for any pupae that have remained in the cups. Most of the pupae will be loose in the bag. The percent efficacy is calculated as follows:

$$\frac{\text{Number of pupae from 4 treatment cups}}{\text{Number of cups/treatment x 20}} \times 100$$

The final results expressed as percent inhibition of pupation are corrected for control mortality by using Abbott's formula. Data obtained are summarized in Table I.

**TABLE I**

**Evaluation Of The Antiparasitic Activity Of Test Compounds**

| Test Compound | *T. colubriformis* (mg/kg) | | | | *P. cuniculi* (micrograms/cm$^2$) | | | | *M. domestica* (ppm) | |
|---|---|---|---|---|---|---|---|---|---|---|
| | 0.125 | 0.0625 | 0.0313 | 0.0156 | 4.0 | 1.0 | 0.1 | 0.01 | 10 | 1.0 |
| 13-Fluoro-23-(O-methyloxime)-LL-F28249alpha | 100 | 100 | 83 | 66 | 100 | 100 | 100 | 96 | 100 | 62 |
| 13-Fluoro-5-(formyloxy)-23-(O-methyloxime)-LL-F28249alpha | 100 | 86 | 82 | - | 100 | 100 | 100 | 38 | - | - |
| 26,27-Epoxy-13-fluoro-23-(O-methyloxime)-LL-F28249alpha | 100 | 100 | 100 | 98 | 100 | 100 | 100 | 7 | 79 | - |
| 5-(Dichloroacetoxy)-13-fluoro-23-(O-methyloxime)-LL-F28249alpha | 100 | 83 | 59 | - | 100 | 100 | 100 | 57 | 15 | - |
| 13-Fluoro-23-(O-methyloxime)-5-(trichloroacetoxy)-LL-F28249alpha | 99 | 77 | 17 | - | 100 | 100 | 100 | 79 | - | - |

**EXAMPLE 19**

**Evaluation Of The Insecticidal And Acaricidal Activity of Test Compounds**

In the following evaluations, test solutions are prepared by dissolving the test compound in a 35% acetone in water mixture to a concentration of 10,000 ppm. Subsequent dilutions are made with water as needed.

**Heliothis virescens, egg, tobacco budworm**

A young cotton leaf about 6-7 cm long is dipped in the test solution with agitation for 3 seconds. Eggs are collected on a cheesecloth and the cheesecloth is cut into 10-20 mm squares containing about 50-100 eggs each (6-30 hours old). A square of cheesecloth with eggs is also dipped in the test solution and placed on the treated leaf. The combination is placed in a hood to dry, then placed in an 8 oz paper cup, into which a 5 cm length of damp dental wick has been placed. A clear plastic lid is put on the top of the cup and the treatments are held for 3 days before mortality counts are made.

**Heliothis virescens, third-instar, tobacco budworm**

Cotton cotyledons are dipped in the test solution and allowed to dry in a hood. When dry, each is cut into quarters and 10 sections are placed, individually, into 30 mL plastic medicine cups containing a 5-7 mm long piece of damp dental wick. One third-instar caterpillar is added to each cup, and a cardboard lid placed on the cup. Treatments are maintained for 3 days before mortality counts and estimates of reduction in feeding damage are made.

**Spondontera eridania, third-instar larvae, Southern armyworm**

A Sieva lima bean leaf expanded to 7-8 cm in length is dipped in the test solution with agitation for 3 seconds and placed in a hood to dry. The leaf is then placed in a 100 x 10 mm petri dish containing a damp filter paper on the bottom and 10 third instar caterpillars. Observations of mortality, reduced feeding, or any interference with normal moulting are made at 3 days and 5 days.

**Aphis fabae, mixed instar, bean aphid**

Pots containing single nasturtium plants (Tropaeolum sp.) about 5 cm tall, are infested with about 100-200 aphids 1 day before the test. Each pot is sprayed with the test solution for 2 revolutions of a 4 rpm turn-table in a hood, using a #154 DeVilbiss atomizer. The spray tip is held about 15 cm from the plant, and the spray directed so as to give complete coverage of the plants and the aphids. The sprayed pots are set on their sides on white enamel trays and held for 2 days, following which mortality estimates are made.

**Empoasca abrupta, adults, western potato leafhopper**

A sieva lima bean leaf about 5 cm long is dipped in the test solution for 3 seconds with agitation and placed in a hood to dry. The leaf is placed in a 100 x 10 mm petri dish containing a moist filter paper on the bottom. About 10 adult leafhoppers are added to each dish, and the treatments are kept for 3 days before mortality counts are made.

**Tetranychus urticae (P-resistant strain) 2-spotted spider mite**

Sieva lima bean plants with primary leaves expanded to 7-8 cm are selected and cut back to one plant per pot. A small piece is cut from a leaf taken from the main colony of mites and placed on each leaf of the test plants. This is done about 2 hours before treatment to allow the mites to move over to the test plant and to lay eggs. The size of the cut piece is varied to obtain about 100 mites per leaf. At the time of the treatment, the piece of leaf used to transfer the mites is removed and discarded. The mite-infested plants are dipped in the test solution for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

| Rating Scale | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

The data obtained are summarized in Table II.

**TABLE II**

**Evaluation Of The Insecticidal And Acaricidal Activity Of Test Compounds**

| Test Compound | Tobacco Budworm egg (ppm) 100 | Tobacco Budworm egg (ppm) 10 | Tobacco Budworm 3rd instar (ppm) 100 | Tobacco Budworm 3rd instar (ppm) 10 | Southern Armyworm Day 3 (ppm) 10 | Southern Armyworm Day 3 (ppm) 1 | Southern Armyworm Day 5 (ppm) 10 | Bean Aphid (ppm) 10 | Leafhopper (ppm) 100 | Leafhopper (ppm) 10 | P-resistant Mites (ppm) 1000 | P-resistant Mites (ppm) 100 | P-resistant Mites (ppm) 10 |
|---|---|---|---|---|---|---|---|---|---|---|---|---|---|
| 13-Fluoro-23-(O-methyloxime)-LL-F28249alpha | 8 | 0 | 9 | 9 | 9 | 4 | 9 | 7 | 7 | 6 | 9 | 5 | 8 |
| 5-(Dichloroacetoxy)-13-fluoro-23-(O-methyloxime)-LL-F28249alpha | 0 | 0 | 9 | 9 | 9 | 5 | 9 | 9 | 7 | 0 | 9 | 9 | 8 |
| 13-Fluoro-23-(O-methyloxime)-5-(trichloroacetoxy)-LL-F28249alpha | 8 | 0 | 9 | 9 | 4 | 3 | 9 | 9 | 0 | 0 | 9 | 9 | 8 |
| 13-Fluoro-5-(formyloxy)-23-(O-methyloxime)-LL-F28249alpha | 0 | 0 | 9 | 8 | 9 | 1 | 9 | 9 | 0 | 0 | 9 | 9 | 8 |

**EXAMPLE 20**

**Preparation of 5-Acetoxy-LL-F28249a**

To a 0°C solution of LL-F28249α (32.57 g), 39.3 mmol) and pyridine (200 mL) is added acetic anhydride (20 mL, 212 mmol). The reaction mixture is stirred for 3 days at 0°C. concentration in vacuo of the reaction mixture yields a yellow residue. The residue is dissolved into ethyl acetate, washed sequentially with water, dilute hydrochloric acid solution, water, 10% sodium bicarbonate solution and brine, dried over anhydrous sodium sulfate and concentrated in vacuo to yield the title compound as a pale yellow foam (35.47 g), identified by NMR spectral analyses.

**EXAMPLE 21**

**Preparation of 5-Acetoxy-23-{[(methoxycarbonyl)carbonyl]oxy}-LL-F28249α**

To a mixture of 5-acetoxy-LL-F28249α (35.47 g, 53.1 mmol), calcium carbonate (11.04 g, 110.3 mmol) and methylene chloride is added methyl oxalyl chloride (8.0 mL, 87 mmol). The reaction mixture is stirred for 2 hours at room temperature. The reaction mixture is then cooled to 0°C and 2 normal hydrochloric acid solution (60 mL) is added slowly to the mixture. Water is added to the mixture and the layers are separated. The organic layer is diluted with methylene chloride, washed sequentially with water, 10% sodium bicarbonate solution, water and brine, dried over anhydrous sodium sulfate and concentrated in vacuo to yield the title compound as a yellow foam (41.28 g), identified by NMR spectral analyses.

**EXAMPLE 22**

**Preparation of 5-Acetoxy-13β-hydroxy-23-{[(methoxycarbonyl)carbonyl]oxy}-LL-F28249α**

To a mixture of 5-acetoxy-23-{[(methoxycarbonyl)carbonyl]oxy}-LL-F28249α (41.28 g, 55.71 mmol) and 88% formic acid solution (200 mL) is added selenium dioxide (8.6 g, 77.5 mmol). The reaction mixture is stirred at room temperature for 90 minutes then the mixture is diluted with ethyl acetate and filtered through diatonaceous earth. The filtrate is cooled in an ice/acetone bath. 20% sodium hydroxide solution (550 mL) is then added slowly with stirring to the cold filtrate. The organic layer is separated and washed sequentially with 10% sodium bicarbonate solution, water and brine, dried over anhydrous sodium sulfate and concentrated in vacuo to yield an orange foam. The foam is dissolved into methanol and 2 normal hydrochloric acid solution (25 mL) is added. The reaction mixture is stirred at room temperature for 2 1/2 hours, then 10% sodium bicarbonate solution (30 mL) is added. Concentration of the mixture in vacuo yields a dark red residue. The red residue is dissolved into ethyl acetate, washed sequentially with water, 10% sodium bicarbonate solution and brine, dried over anhydrous sodium sulfate and concentrated in vacuo to yield an orange foam. The foam is chromatographed using silica gel and hexanes/ethyl acetate (1:1) as eluant to yield the title compound as an off-white powder (11.50 g), identified by NMR spectral analyses.

**EXAMPLE 23**

**Preparation of 5-Acetoxy-13β-[(ethoxycarbonyl)oxy]-23-{[(methoxycarbonyl)carbonyl]oxy}-LL-F28249α**

A mixture of 5-acetoxy-13β-hydroxy-23-{[(methoxycarbonyl)carbonyl]oxy}-LL-F28249α (0.51 g, 0.67 mmol) in methylene chloride, under nitrogen, is treated with ethyl chloroformate (0.23 g, 2.12 mmol), pyridine (0.25 g, 3.16 mmol) and 4-dimethylaminopyridine (0.05 g, 0.41 mmol) at room temperature. Stirring is continued at room temperature for two hours then the reaction mixture is diluted with ethyl acetate, washed sequentially with water, 5% hydrochloric acid solution, water, 10% sodium bicarbonate solution and brine, dried over anhydrous sodium sulfate and concentrated in vacuo to give a residue. The residue is chromatographed using silica gel and eluted with hexanes/ethyl acetate (3:1) to yield the title compound as a white powder (0.33 g, 59%), identified by $^{1}$HNMR $^{13}$CNMR and mass spectral analyses.

**EXAMPLE 24**

**Preparation of 5-Acetoxy-13β-methyl-LL-F28249α**

A -78°C mixture of 5-acetoxy-13β-[(ethoxycarbonyl)oxy]-23-{[(methoxycarbonyl)carbonyl]oxy}-LL-F28249α (0.26 g, 0.31 mmol) in methylene chloride, under nitrogen, is treated over a two minute period with trimethylaluminum (0.46 g, 6.4 mmol). Stirring is continued for 15 minutes at -78°C, then the reaction mixture is warmed to 0°C and kept at 0°C with an ice-bath for 30 minutes, the ice-bath is removed and the reaction mixture is stirred at room temperature for 4 hours.

While cooling with an ice-bath, water (0.5 mL) is slowly added to the reaction mixture. After the gas evolution is complete, the reaction mixture is poured into a mixture of ethyl acetate and 2 normal hydrochloric acid solution. The ethyl acetate layer is separated and washed sequentially with water, 10% sodium bicarbonate solution and brine, dried over anhydrous sodium sulfate and concentrated in vacuo to yield a residue. The residue is chromatographed using silica gel and methylene chloride/acetonitrile (9:1) as eluant to yield the title compound as a white powder (0.144 g, 56%), identified by $^1$HNMR, $^{13}$CNMR and mass spectral analyses.

Following the procedure of Example 5, but substituting triethylaluminum for trimethylaluminum yields 5-acetoxy-13β-ethyl-LL-F28249α.

**EXAMPLE 25**

**Preparation of 5-Acetoxy-13β-methyl-23-oxo-LL-F28249α**

To a mixture of 5-acetoxy-13β-methyl-LL-F28249a (0.188 g, 0.28 mmol), a few 4 angstrom molecular sieves and acetonitrile is added 4-methylmorpholine N-oxide (0.131 g, 1.12 mmol). After stirring for 15 minutes at room temperature tetrapropylammonium perruthenate (0.047 g, 0.13 mmol) is added to the mixture and stirring is continued for one hour. The reaction mixture is filtered through diatomaceous earth, diluted with ethyl acetate and washed sequentially with water, dilute hydrochloric acid solution, water, 10% sodium bicarbonate solution and brine, dried over anhydrous sodium sulfate and concentrated in vacuo to yield a residue. The residue is chromatographed using silica gel and methylene chloride/acetonitrile (19:1) as eluant to yield the title compound as a white powder (0.108 g, 57%), identified by $^1$HNMR, $^{13}$CNMR and mass spectral analyses.

Following the procedure of Example 6, but using 5-acetoxy-13β-ethyl-LL-F28249α for 5-acetoxy-13β-methyl-LL-F28249αyields 5-acetoxy-13β-ethyl-23-oxo-LL-F28249α.

**EXAMPLE 26**

**Preparation of 5-Acetoxy-13β-methyl-23-(O-methyloxime)-LL-F28249α**

A mixture of 5-acetoxy-13β-methyl-23-oxo-LL-F28249α (0.144 g, 0.22 mmol), sodium acetate (0.096 g, 1.17 mmol), methoxylamine hydrochloride (0.092 g, 1.1 mmol) and methanol is stirred for one hour at room temperature. The reaction mixture is diluted with ethyl acetate, washed sequentially with water and brine, dried over anhydrous sodium sulfate and concentrated in vacuo to yield a residue. The residue is chromatographed using silica gel and hexanes/ethyl acetate (4:1) as eluant to yield the title compound as a white solid (0.125 g, 83%), identified by $^1$HNMR, $^{13}$CNMR and mass spectral analyses.

Following the procedure of Example 7, substituting 5-acetoxy-13β-ethyl-23-oxo-LL-F28249α for 5-acetoxy-13β-methyl-23-oxo-LL-F28249α yields 5-acetoxy-13β-ethyl-23-(O-methyloxime)-LL-F28249α.

**EXAMPLE 27**

**Preparation of 13β-Methyl-23-(O-methyloxime)-LL-F28249α**

To a 0°C solution of 5-acetoxy-13β-methyl-23-(O-methyloxime)-LL-F28249α (0.11 g, 0.16 mmol) and methanol is added sodium hydroxide (0.5 mL, 0.50 mmol). Stirring is continued at 0°C for two hours, then the reaction mixture is diluted with ethyl acetate, washed sequentially with water and brine, dried over anhydrous sodium sulfate and concentrated in vacuo to yield a residue. The residue is chromatographed using silica gel and hexanes/ethyl acetate (3:1) as eluant to yield the title compound as a white foam (0.079 g, 77%), identified by $^1$HNMR, $^{13}$CNMR and mass spectral analyses.

Following the procedure of Example 0, using 5-acetoxy-13β-ethyl-23-(O-methyloxime)-LL-F28249α in place of 5-acetoxy-13β-methyl-23-(O-methyloxime)-LL-F28249α yields 13β-ethyl-23-(O-methyloxime)-LL-F28249α.

## EXAMPLE 28

### Evaluation Of The Antiparasitic Activity Of Test Compounds.

The antiparasitic activity of the compounds of the present invention at various concentrations of active ingredient is determined by the following test examples. The results of these tests are summarized in Table I.

### Evaluation of test compounds for controlling Trichostrongylus colubriformis in warm-blooded animals

In these tests the active ingredient is dissolved in polyethylene glycol and dimethylsulfoxide (PEG:DMSO) (1:2 v/v) in sufficient quantity to provide treatments that deliver from 0.0156 to 0.1250 mg/kg of test compound to the animal.

To evaluate the test compounds, 5 week old male gerbils are infected with 400-600 infective T. colubriformis larvae of sheep origin on day 0. On day 7, the gerbils are weighed and treatment initiated. Test compounds are administered by gavage on the 7th day after treatment. The gerbils are sacrificed on the 11th day after treatment, and the remaining worms counted. The percent efficacy is calculated by comparing worm counts in treated animals with those from untreated infected controls using the following formula.

$$\frac{\text{Control mean} - \text{treated mean}}{\text{control mean}} \times 100 = \%\ \text{Efficacy}$$

Three replicates per treatment are employed in these evaluations.

The data obtained are summarized in Table I.

### Evaluation of test compounds for controlling Psoroptes cuniculi (Ear Mites)

On the day prior to test, or the morning of test, test compounds are dissolved in acetone and diluted to the desired concentrations. The concentration should be calculated so that 400 µL contains the amount to be placed on each filter paper. 400 µL of this solution is pipetted onto a top (3.7 cm dia) and bottom (3.5 cm dia) filter paper disc which is then placed on a ceramic plate to dry. [NOTE: This should be done under a hood.] There is a rough and smooth side to the filter paper. The test solution should be applied to the rough side which is placed up while drying. When dry, the two discs are placed in a Petri dish with the rough sides facing in separated by a small piece of stiff paper folded in the shape of a tent. Dishes are held at room temperature overnight, if done the day before the test. A standard at 0.01, 0.1 and 1.0 µL/$cm^2$ is run in each test.

Scab (containing mites) is collected from the ears of infested rabbits the morning of the test. This material is placed in a large Petri dish under an illuminated magnifier. Mites crawl out of the scab and are easily collected on the point of a dissecting needle or one prong of a pair of fine forceps. The top filter paper in each dish is removed and 12 mites are placed on the bottom disk and the top paper replaced. Before replacing the top of the Petri dish the rim of the dish is smeared with Vaseline to trap any escaping mites.

For evaluation tests there are generally 4 replicates of each dose which are counted, 2 at 4 hours and 2 at 24 hours.

After mites are added to the dishes, the dishes in each replicate are placed in a tray which is then placed in a plastic bag with several wet towels and held at room temperature.

After 4 or 24 hours, dishes are examined under a dissecting scope as follows -

1. Open dish carefully, remove top filter paper and save.
2. Draw a small circle approximately 1/2 cm in diameter on bottom filter paper using a soft pencil.

3. Using a disposable pipette, gently wet the area in and around the circle.
4. Transfer all mites from the dish into this circle. Look carefully - on cover, top filter paper and under bottom paper for mites.
5. Count and record the number of mites in the circle.
6. Replace the top cover and set the dish aside.
7. A minimum of 15 minutes later, count the mites remaining in the circle (these are dead mites).
8. Calculate and record the number of live mites.
9. Calculate percent efficacy as follows:

$$\frac{\text{Total of Dead mites}}{\text{Total number of mites}} \times 100 = \%\ \text{Efficacy}$$

The data obtained are summarized in Table III.

TABLE III.

**Evaluation Of The Antiparasitic Activity Of Test Compounds**

| Test Compound | *T. colubriformis* (mg/kg) | | | | *P. cuniculi* (micrograms/$cm^2$) | | | |
|---|---|---|---|---|---|---|---|---|
| | 0.125 | 0.0625 | 0.0313 | 0.0156 | 4.0 | 1.0 | 0.1 | 0.01 |
| 13beta-Methyl-23-(O-methyloxime)-LL-F28249alpha | 99 | 98 | 53 | 17 | 100 | 98 | 98 | 100 |
| 13beta-Ethyl-23-(O-methyloxime)-LL-F28249alpha | 95 | | | | 91 | | | |

**EXAMPLE 29**

**Evaluation Of The Insecticidal And Acaricidal Activity of Test Compounds**

In the following evaluations, test solutions are prepared by dissolving the test compound in a 35% acetone in water mixture to a concentration of 10,000 ppm. Subsequent dilutions are made with water as needed.

**Heliothis virescens, egg, tobacco budworm**

A young cotton leaf about 6-7 cm long is dipped in the test solution with agitation for 3 seconds. Eggs are collected on a cheesecloth and the cheesecloth is cut into 10-20 mm squares containing about 50-100 eggs each (6-30 hours old). A square of cheesecloth with eggs is also dipped in the test solution and placed on the treated leaf. The combination is placed in a hood to dry, then placed in an 8 oz paper cup, into which a 5 cm length of damp dental wick has been placed. A clear plastic lid is put on the top of the cup and the treatments are held for 3 days before mortality counts are made.

**Aphis fabae, mixed instar, bean aphid**

Pots containing single nasturtium plants (Tropaeolum sp.) about 5 cm tall, are infested with about 100-200 aphids 1 day before the test. Each pot is sprayed with the test solution for 2 revolutions of a 4 rpm turn-table in a hood, using a #154 DeVilbiss atomizer. The spray tip is held about 15 cm from the plant, and the spray directed so as to give complete coverage of the plants and the aphids. The sprayed pots are set on their sides on white enamel trays and held for 2 days, following which mortality estimates are made.

**Tetranychus urticae (P-resistant strain) 2-spotted spider mite**

Sieva lima bean plants with primary leaves expanded to 7-8 cm are selected and cut back to one plant per pot. A small piece is cut from a leaf taken from the main colony of mites and placed on each leaf of the test plants. This is done about 2 hours before treatment to allow the mites to move over to the test plant and to lay eggs. The size of the cut piece is varied to obtain about 100 mites per leaf. At the time of the treatment, the piece of leaf used to transfer the mites is removed and discarded. The mite-infested plants are dipped in the test solution for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

| Rating Scale | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

The data obtained are summarized in Table IV.

TABLE IV

Evaluation Of The Insecticidal And Acaricidal Activity Of Test Compounds

| Test Compound | Tobacco Budworm egg (ppm) | | Bean Aphid (ppm) | P-resistant Mites (ppm) | |
|---|---|---|---|---|---|
| | 100 | 10 | 10 | 1 | 0.1 |
| 13beta-Methyl-23-(O-methyloxime)-LL-F28249alpha | 8 | 8 | 9 | 9 | 9 |
| 13beta-Ethyl-23-(O-methyloxime)-LL-F28249alpha | 9 | 9 | 5 | 9 | 8 |

## Claims

**Claims for the following Contracting States : AT, BE, CH, DE, DK, FR, GB, IT, LI, LU, NL, SE**

**1.** A compound having the structural formula:

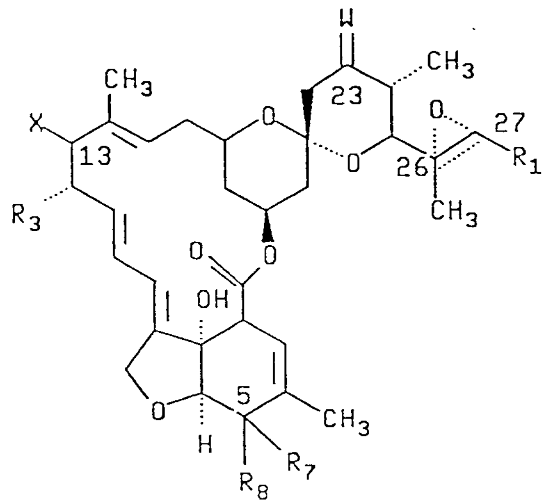

wherein

- $R_1$ is methyl, ethyl or isopropyl;
- $R_7$ is hydrogen and $R_8$ is $OR_2$, or when taken together with the carbon atom to which they are attached $R_7$ and $R_8$ represent C=0;
- $R_2$ is hydrogen, $C_1$-$C_4$ alkyl, or

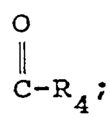

- $R_4$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy;
- $R_3$ is hydrogen, methyl or ethyl;
- X is fluorine, bromine, chlorine or iodine;
- W is =N-Y ;
- Y is $OR_5$ or $NHR_6$;
- $R_5$ is $C_1$-$C_4$ alkyl or $C_1$-$C_4$ acyl;
- $R_6$ is $C_1$-$C_4$ acyl; and

the dotted triangular figure with oxygen at $C_{26}$-$C_{27}$ indicates that there is present either a double bond or an epoxide.

**2.** The compound according to claim 1, wherein $R_1$ is isopropyl;

- $R_7$ is hydrogen and $R_8$ is $OR_2$;
- $R_2$ is hydrogen, $C_1$-$C_4$ alkyl, or

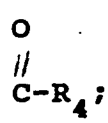

$R_4$ is hydrogen, methyl, chloromethyl, dichloromethyl, trichloromethyl, or methoxymethyl;
$R_3$ is methyl;
X is fluorine;
W is N-Y;
Y is $OR_5$; and
$R_5$ is $C_1$-$C_4$ alkyl.

3. A method for protecting crops, trees, shrubs, stored grain and ornamental plants from attack by an insect, acarid and nematode characterized by applying to said crops, trees, shrubs, stored grain and ornamental plants an insecticidally, acaricidally or nematicidally effective amount of a compound having the structural formula in Claim 1.

4. The method according to claim 3, wherein the compound has the structural formula as described in claim 6 and
$R_1$ is isopropyl;
$R_7$ is hydrogen; $R_8$ is $OR_2$;
$R_2$ is hydrogen, $C_1$-$C_4$ alkyl, or

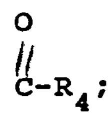

$R_4$ is hydrogen, methyl, chloromethyl, dichloromethyl, trichloromethyl, or methoxymethyl;
$R_3$ is methyl;
X is fluorine;
W is N-Y;
Y is $OR_5$; and
$R_5$ is $C_1$-$C_4$ alkyl.

5. A composition for controlling endo- and ectoparasites, insects, acarids and nematodes characterized by an agronomically acceptable carrier containing a prophylactically, therapeutically, pharmaceutically or insecticidally effective amount of a compound having the structural formula in Claim 1.

6. A compound having the structural formula:

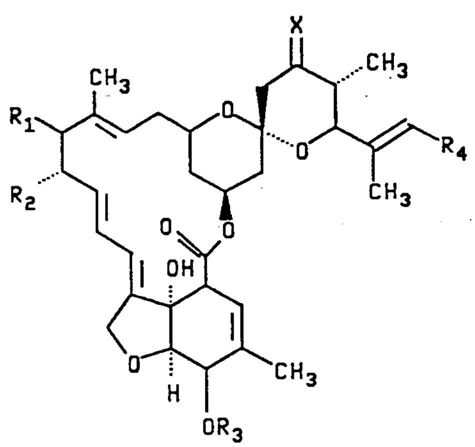

(I)

wherein
$R_1$ is $C_1$-$C_4$ alkyl;

$R_2$ is hydrogen, methyl or ethyl;
$R_3$ is hydrogen, $C_1$-$C_4$ alkyl or

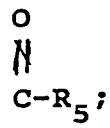

$R_5$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy;
$R_4$ is methyl, ethyl or isopropyl;
X is oxygen, $NOR_6$ or N-$NHR_7$;
$R_6$ is hydrogen, $C_1$-$C_4$ alkyl, $C_1$-$C_4$ alkoxymethyl, $C_1$-$C_4$ alkanoyl, benzyl or phenyl; and
$R_7$ is $C_1$-$C_4$ acyl, $C_1$-$C_4$ alkyl or benzoyl.

**7.** The compound according to Claim 6, wherein
$R_1$ is methyl, ethyl or isopropyl;
$R_2$ is methyl;
$R_3$ is hydrogen, $C_1$-$C_4$ alkyl or

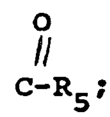

$R_5$ is hydrogen, $C_1$-$C_4$ alkoxymethyl, $C_1$-$C_4$ halomethyl or formyl;
$R_4$ is isopropyl;
X is $NOR_6$; and
$R_6$ is $C_1$-$C_4$ alkyl.

**8.** A composition for controlling endo- and ectoparasites, insects, acarids and nematodes characterized by an agronomically acceptable carrier containing a prophylactically, therapeutically, pharmaceutically or insecticidally effective amount of the compound of claim 6.

**Claims for the following Contracting States : ES, GR**

**1.** A process for producing 13-halo-23-imino derivatives of LL-F28249 compounds of general formula

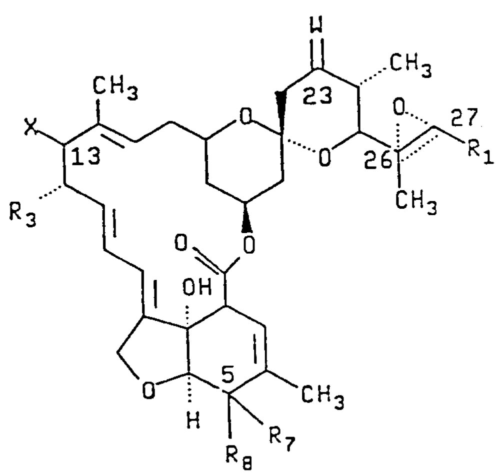

wherein

- $R_1$ is methyl, ethyl or isopropyl;
- $R_7$ is hydrogen and $R_8$ is $OR_2$, or when taken together with the carbon atom to which they are attached $R_7$ and $R_8$ represent C=O;
- $R_2$ is hydrogen, $C_1$-$C_4$ alkyl, or

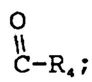

- $R_4$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy;
- $R_3$ is hydrogen, methyl or ethyl;
- X is fluorine, bromine, chlorine or iodine;
- W is =N-Y;
- Y is $OR_5$ or $NHR_6$;
- $R_5$ is $C_1$-$C_4$ alkyl or $C_1$-$C_4$ acyl;
- $R_6$ is $C_1$-$C_4$ acyl; and

the dotted triangular figure with oxygen at $C_{26}$-$C_{27}$ indicates that there is present either a double bond or an epoxide, which comprises:

A) reacting a 13-hydroxy-5,23-dioxo-LL-F28249 compound with a halogenating reagent to render the corresponding 13-halo-5,23-dioxo-LL-F28249 compound which is reacted sequentially with an alkoxylamine or with hydroxylamine and sodium borohydride to render the corresponding 13-halo-23-imino-LL-F28249 compound; or alternatively,

B) reacting a 5-hydroxy-13-halo-23-imino-LL-F28249 compound with an alkylating or acylating reagent to render the corresponding 5-alkyloxy or 5-acyloxy-13-halo-23-imino-LL-F28249 compound; or alternatively,

C) reacting a 13-hydroxy-23-oxo-LL-F28249 compound with a halogenating reagent, followed by the reaction with an alkoxylamine or with hydroxylamine to render the corresponding 13-halo-23-imino-5-acyloxy-LL-F28249 compound, which may be, if desired, hydrolyzed in the presence of a base to render the corresponding 13-halo-23-imino-LL-F28249 compound; or alternatively,

D) reacting a 13-halo-23-oxo-LL-F28249 compound with m-chloroperbenzoic acid to render the corresponding 13-halo-23-oxo-26,27-epoxide-LL-F28249compound which is further reacted with an alkoxyl, acyloxyl or hydroxyl oxime to render the corresponding 23-(O-alkyloxime)-or 23-(O-acyloxime)- or 23-oxime- derivatives of 13-halo-26,27-epoxide-LL-F28249 compounds.

**2.** A process according to claim 1, wherein in the compound obtained:

- $R_1$ is isopropyl;
- $R_7$ is hydrogen and $R_8$ is $OR_2$;
- $R_2$ is hydrogen, $C_1$-$C_4$ alkyl, or

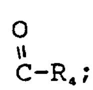

- $R_4$ is hydrogen, methyl, chloromethyl, dichloromethyl, trichloromethyl or methoxymethyl;
- $R_3$ is methyl;
- X is fluorine;
- W is =N-Y;
- Y is $OR_5$; and
- $R_5$ is $C_1$-$C_4$ alkyl.

**3.** A process for producing 13-alkyl-23-imino derivatives of LL-F28249 compounds of general formula

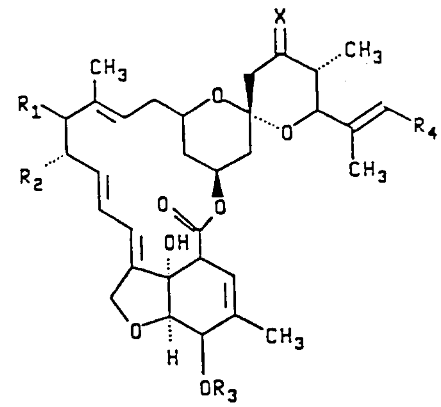

(I)

wherein

$R_1$ is $C_1$-$C_4$ alkyl;

$R_2$ is hydrogen, methyl or ethyl;

$R_3$ is hydrogen, $C_1$-$C_4$ alkyl, or

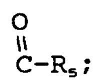

$R_5$ is hydrogen, $C_1$-$C_4$ alkyl, $C_1$-$C_4$ haloalkyl, $C_1$-$C_4$ alkoxy, phenoxy, $C_1$-$C_4$ alkoxymethyl, phenoxymethyl, or phenyl optionally substituted with 1 nitro, 1-3 halogens, 1-3 $C_1$-$C_4$ alkyl, or 1-3 $C_1$-$C_4$ alkoxy;

$R_4$ is methyl, ethyl or isopropyl;

X is oxygen, $NOR_6$ or $N$-$NHR_7$;

$R_6$ is hydrogen, $C_1$-$C_4$ alkyl, $C_1$-$C_4$ alkoxymethyl, $C_1$-$C_4$ alkanoyl, benzyl or phenyl; and

$R_7$ is $C_1$-$C_4$ acyl, $C_1$-$C_4$ alkyl or benzoyl;

which comprises:

reacting a 5-acetoxy-13$\beta$-[ethoxycarbonyl)oxy]-2-{[(methoxycarbonyl)carbonyl]oxy}-LL-F28249 compound with a trialkylaluminum to render the corresponding 5-acetoxy-13$\beta$-alkyl-LL-F28249 compound which is reacted with 4-methylmorpholine N-oxide and tetrapropylammonium perruthenate to render the corresponding 5-acetoxy-13$\beta$-alkyl-23-oxo-LL-F28249, followed, if desired, by treatment with an alkoxylamine hydrochloride to render the corresponding 5-acetoxy-13$\beta$-alkyl-23-(O-alkyloxime)-LL-F28249 compound, which may be, if desired, hydrolyzed with a strong base to render the corresponding 13$\beta$-alkyl-23-(O-alkyloxime)-LL-F28249 compound of the formula (I) previously shown.

**4.** A process according to claim 3, wherein in the compound obtained:

$R_1$ is methyl, ethyl or isopropyl;

$R_2$ is methyl;

$R_3$ is hydrogen, $C_1$-$C_4$ alkyl, or

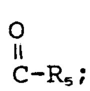

$R_5$ is hydrogen, $C_1$-$C_4$ alkoxymethyl, $C_1$-$C_4$ halomethyl or formyl;

$R_4$ is isopropyl;

X is $NOR_6$; and

$R_6$ is $C_1$-$C_4$ alkyl.

5. A method for protecting crops, trees, shrubs, stored grain, and ornamental plants from attack by an insect, acarid and nematode characterized by applying to said crops, trees, shrubs, stored grain, and ornamental plants an insecticidally, acaricidally or nematicidally effective amount of a compound obtainable according to the process of claim 1.

6. A method according to claim 5, wherein the compound has the structural formula shown in claim 1 and
$R_1$ is isopropyl;
$R_7$ is hydrogen and $R_8$ is $OR_2$;
$R_2$ is hydrogen, $C_1$-$C_4$ alkyl, or

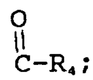

$R_4$ is hydrogen, methyl, chloromethyl, dichloromethyl, trichloromethyl or methoxymethyl;
$R_3$ is methyl;
X is fluorine;
W is =N-Y;
Y is $OR_5$; and
$R_5$ is $C_1$-$C_4$ alkyl.

## Patentansprüche
## Patentansprüche für folgende Vertragsstaaten : AT, BE, CH, DE, DK, FR, GB, IT, LI, LU, NL, SE

1. Verbindung mit der Strukturformel:

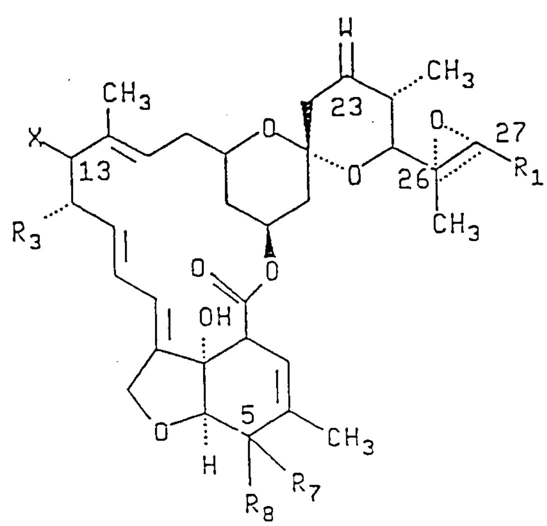

in der
$R_1$ Methyl, Ethyl oder Isopropyl ist;
$R_7$ Wasserstoff ist und $R_8$ $OR_2$ ist, oder, wenn sie zusammen mit dem Kohlenstoffatom genommen werden, mit dem sie verknüpft sind, $R_7$ und $R_8$ C=O darstellen;
$R_2$ Wasserstoff, ($C_1$-$C_4$)-Alkyl oder

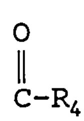

ist;
$R_4$ Wasserstoff, $(C_1-C_4)$-Alkyl, $(C_1-C_4)$-Halogenalkyl, $(C_1-C_4)$-Alkoxy, Phenoxy, $(C_1-C_4)$-Alkoxymethyl, Phenoxymethyl oder Phenyl, gegebenenfalls mit 1 Nitro, 1-3 Halogenen, 1-3 $(C_1-C_4)$ -Alkyl oder 1-3 $(C_1-C_4)$-Alkoxy substituiert, ist;
$R_3$ Wasserstoff, Methyl oder Ethyl ist;
X Fluor, Brom, Chlor oder Iod ist;
W =N-Y ist;
Y $OR_5$ oder $NHR_6$ ist;
$R_5$ $(C_1-C_4)$-Alkyl oder $(C_1-C_4)$-Acyl ist;
$R_6$ $(C_1-C_4)$-Acyl ist; und
die gepunktete dreieckige Figur mit Sauerstoff an $C_{26}$-$C_{27}$ anzeigt, daß an dieser Stelle entweder eine Doppelbindung oder ein Epoxid vorhanden ist.

**2.** Verbindung nach Anspruch 1, in der $R_1$ Isopropyl ist;
$R_7$ Wasserstoff ist und $R_8$ $OR_2$ ist;
$R_2$ Wasserstoff, $(C_1-C_4)$-Alkyl oder

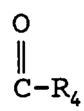

ist;
$R_4$ Wasserstoff, Methyl, Chlormethyl, Dichlormethyl, Trichlormethyl oder Methoxymethyl ist;
$R_3$ Methyl ist;
X Fluor ist;
W N-Y ist;
Y $OR_5$ ist; und
$R_5$ $(C_1-C_4)$-Alkyl ist.

**3.** Verfahren zum Schützen von Feldfrüchten, Bäumen, Sträuchern, gelagertem Korn und Zierpflanzen vor dem Angriff durch ein Insekt, eine Milbe und einen Fadenwurm, dadurch gekennzeichnet, daß man auf die Feldfrüchte, Bäume, Sträucher, das gelagerte Korn und die Zierpflanzen eine insektizid, akarizid oder nematizid wirksame Menge einer Verbindung mit der Strukturformel nach Anspruch 1 anwendet.

**4.** Verfahren nach Anspruch 3, in dem die Verbindung die in Anspruch 6 beschriebene Strukturformel aufweist und
$R_1$ Isopropyl ist;
$R_7$ Wasserstoff ist und $R_8$ $OR_2$ ist;
$R_2$ Wasserstoff, $(C_1-C_4)$-Alkyl oder

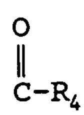

ist;
$R_4$ Wasserstoff, Methyl, Chlormethyl, Dichlormethyl, Trichlormethyl oder Methoxymethyl ist;
$R_3$ Methyl ist;
X Fluor ist;
W N-Y ist;
Y $OR_5$ ist; und
$R_5$ $(C_1-C_4)$-Alkyl ist.

**5.** Zusammensetzung zur Kontrolle von Endo- und Ektoparasiten, Insekten, Milben und Fadenwürmern, gekennzeichnet durch einen landwirtschaftlich annehmbaren Träger, der eine prophylaktisch, therapeutisch, pharmazeutisch oder insektizid wirksame Menge einer Verbindung mit der Strukturformel in

Anspruch 1 enthält.

6. Verbindung mit der Strukturformel:

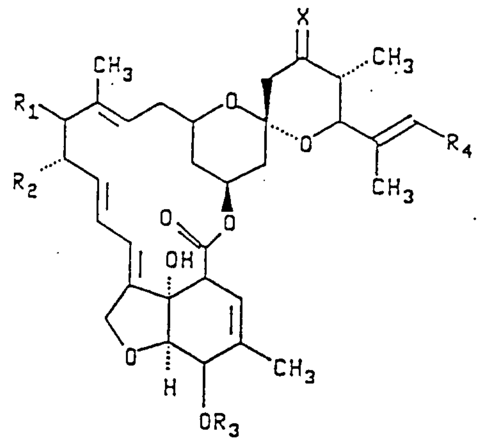

(I)

in der
$R_1$ $(C_1\text{-}C_4)$-Alkyl ist;
$R_2$ Wasserstoff, Methyl oder Ethyl ist;
$R_3$ Wasserstoff, $(C_1\text{-}C_4)$-Alkyl oder

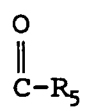

ist;
$R_5$ Wasserstoff, $(C_1\text{-}C_4)$-Alkyl, $(C_1\text{-}C_4)$-Halogenalkyl, $(C_1\text{-}C_4)$-Alkoxy, Phenoxy, $(C_1\text{-}C_4)$-Alkoxymethyl, Phenoxymethyl oder Phenyl, gegebenenfalls mit 1 Nitro, 1-3 Halogenen, 1-3 $(C_1\text{-}C_4)$-Alkyl oder 1-3 $(C_1\text{-}C_4)$-Alkoxy substituiert, ist;
$R_4$ Methyl, Ethyl oder Isopropyl ist;
X Sauerstoff, $NOR_6$ oder $N\text{-}NHR_7$ ist;
$R_6$ Wasserstoff, $(C_1\text{-}C_4)$-Alkyl, $(C_1\text{-}C_4)$-Alkoxymethyl, $(C_1\text{-}C_4)$-Alkanoyl, Benzyl oder Phenyl ist;
$R_7$ $(C_1\text{-}C_4)$-Acyl, $(C_1\text{-}C_4)$-Alkyl oder Benzoyl ist.

7. Verbindung nach Anspruch 6, in der
$R_1$ Methyl, Ethyl oder Isopropyl ist;
$R_2$ Methyl ist;
$R_3$ Wasserstoff, $(C_1\text{-}C_4)$-Alkyl oder

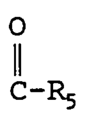

ist;
$R_5$ Wasserstoff, $(C_1\text{-}C_4)$-Alkoxymethyl, $(C_1\text{-}C_4)$-Halogenmethyl oder Formyl ist;
$R_4$ Isopropyl ist;
X $NOR_6$ ist; und

$R_6$ ($C_1$-$C_4$)-Alkyl ist.

**8.** Zusammensetzung zur Kontrolle von Endo- und Ektoparasiten, Insekten, Milben und Fadenwürmern, gekennzeichnet durch einen landwirtschaftlich annehmbaren Träger, der eine prophylaktisch, therapeutisch, pharmazeutisch oder insektizid wirksame Menge einer Verbindung mit der Strukturformel nach Anspruch 6 enthält.

**Patentansprüche für folgende Vertragsstaaten : ES, GR**

**1.** Verfahren zur Herstellung von 13-Halogen-23-imino-Derivaten von LL-F28249-Verbindungen der allgemeinen Formel

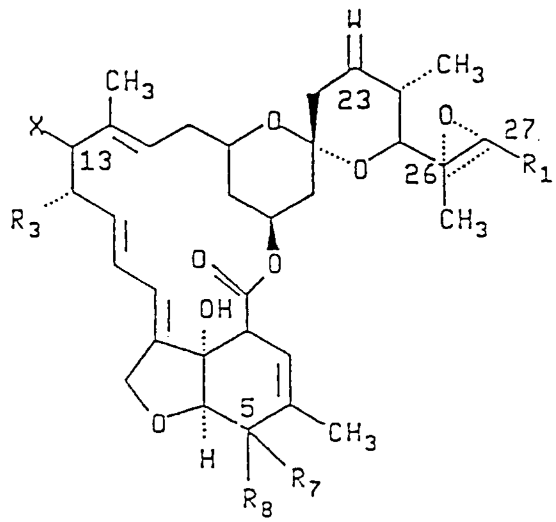

in der

$R_1$ Methyl, Ethyl oder Isopropyl ist;

$R_7$ Wasserstoff ist und $R_8OR_2$ ist, oder, wenn sie zusammen mit dem Kohlenstoffatom genommen werden, mit dem sie verknüpft sind, $R_7$ und $R_8$ C = O darstellen;

$R_2$ Wasserstoff, ($C_1$-$C_4$)-Alkyl oder

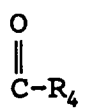

ist;

$R_4$ Wasserstoff, ($C_1$-$C_4$)-Alkyl, ($C_1$-$C_4$)-Halogenalkyl, ($C_1$-$C_4$)-Alkoxy, Phenoxy, ($C_1$-$C_4$)-Alkoxymethyl, Phenoxymethyl oder Phenyl, gegebenenfalls mit 1 Nitro, 1-3 Halogenen, 1-3 ($C_1$-$C_4$)-Alkyl oder 1-3 ($C_1$-$C_4$)-Alkoxy substituiert, ist;

$R_3$ Wasserstoff, Methyl oder Ethyl ist;

X Fluor, Brom, Chlor oder Iod ist;

W = N-Y ist;

Y $OR_5$ oder $NHR_6$ ist;

$R_5$ ($C_1$-$C_4$)-Alkyl oder ($C_1$-$C_4$)-Acyl ist;

$R_6$ ($C_1$-$C_4$)-Acyl ist; und

die gepunktete dreieckige Figur mit Sauerstoff an $C_{26}$-$C_{27}$ anzeigt, daß an dieser Stelle entweder eine Doppelbindung oder ein Epoxid vorhanden ist,

umfassend:

(A) das Umsetzen einer 13-Hydroxy-5,23-dioxo-LL-F28249-Verbindung mit einem Halogenierungsreagenz, um die entsprechende 13-Halogen-5,23-dioxo-LL-F28249-Verbindung zu liefern, die nacheinander mit einem Alkoxylamin oder mit Hydroxylamin und Natriumborhydrid umgesetzt wird, um

die entsprechende 13-Halogen-23-imino-LL-F28249-Verbindung zu liefern; oder alternativ

(B) das Umsetzen einer 5-Hydroxy-13-halogen-23-imino-LL-F28249-Verbindung mit einem Alkylierungs- oder Acylierungsreagenz, um die entsprechende 5-Alkyloxy- oder 5-Acyloxy-13-halogen-23-imino-LL-F28249-Verbindung zu liefern; oder alternativ

(C) das Umsetzen einer 13-Hydroxy-23-oxo-LL-F28249-Verbindung mit einem Halogenierungsreagenz, gefolgt von der Umsetzung mit einem Alkoxylamin oder mit Hydroxylamin, um die entsprechende 13-Halogen-23-imino-5-acyloxy-LL-F28249-Verbindung zu liefern, die, falls gewünscht, in Anwesenheit einer Base hydrolysiert werden kann, um die entsprechende 13-Halogen-23-imino-LL-F28249-Verbindung zu liefern; oder alternativ

(D) das Umsetzen einer 13-Halogen-23-oxo-LL-F28249-Verbindung mit m-Chlorperbenzoesäure, um die entsprechende 13-Halogen-23-oxo-26,27-epoxid-LL-F28249-Verbindung zu liefern, die weiter mit einem Alkoxyl-, Acyloxyl- oder Hydroxyloxim umgesetzt wird, um die entsprechende 23-(O-Alkyloxim)- oder 23-(O-Acyloxim)- oder 23-Oxim-Derivate von 13-Halogen-26,27-epoxid-LL-F28249-Verbindungen zu liefern.

**2.** Verfahren nach Anspruch 1, worin in der erhaltenen Verbindung
$R_1$ Isopropyl ist;
$R_7$ Wasserstoff ist und $R_8$ $OR_2$ ist;
$R_2$ Wasserstoff, $(C_1-C_4)$-Alkyl oder

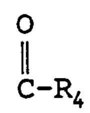

ist;
$R_4$ Wasserstoff, Methyl, Chlormethyl, Dichlormethyl, Trichlormethyl oder Methoxymethyl ist;
$R_3$ Methyl ist;
X Fluor ist;
W =N-Y ist;
Y $OR_5$ ist; und
$R_5$ $(C_1-C_4)$-Alkyl ist.

**3.** Verfahren zur Herstellung von 13-Alkyl-23-imino-Derivaten von LL-F28249-Verbindungen der allgemeinen Formel

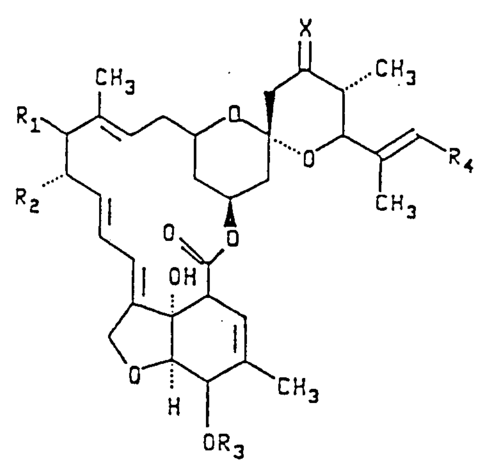

(I)

in der
$R_1$ $(C_1-C_4)$-Alkyl ist;
$R_2$ Wasserstoff, Methyl oder Ethyl ist;

$R_3$ Wasserstoff, ($C_1$-$C_4$)-Alkyl oder

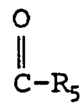

ist;
$R_5$ Wasserstoff, ($C_1$-$C_4$)-Alkyl, ($C_1$-$C_4$)-Halogenalkyl, ($C_1$-$C_4$)-Alkoxy, Phenoxy, ($C_1$-$C_4$)-Alkoxymethyl, Phenoxymethyl oder Phenyl, gegebenenfalls mit 1 Nitro, 1-3 Halogenen, 1-3 ($C_1$-$C_4$)-Alkyl oder 1-3 ($C_1$-$C_4$)-Alkoxy substituiert, ist;
$R_4$ Methyl, Ethyl oder Isopropyl ist;
X Sauerstoff, $NOR_6$ oder N-$NHR_7$ ist;
$R_6$ Wasserstoff, ($C_1$-$C_4$)-Alkyl, ($C_1$-$C_4$)-Alkoxymethyl, ($C_1$-$C_4$)-Alkanoyl, Benzyl oder Phenyl ist;
$R_7$ ($C_1$-$C_4$)-Acyl, ($C_1$-$C_4$)-Alkyl oder Benzoyl ist;
umfassend:
das Umsetzen einer 5-Acetoxy-13β-[(ethoxycarbonyl)oxy]-2-{[(methoxycarbonyl)carbonyl]oxy}-LL-F28249-Verbindung mit einem Trialkylaluminium, um die entsprechende 5-Acetoxy-13β-alkyl-LL-F28249-Verbindung zu liefern, die mit 4-Methylmorpholin-N-oxid und Tetrapropylammoniumperruthenat umgesetzt wird, um die entsprechende 5-Acetoxy-13β-alkyl-23-oxo-LL-F28249-Verbindung zu liefern, gefolgt, falls gewünscht, von der Behandlung mit einem Alkoxylaminhydrochlorid, um die entsprechende 5-Acetoxy-13β-alkyl-23-(O-alkyloxim)-LL-F28249-Verbindung zu liefern, die, falls gewünscht, mit einer starken Base hydrolysiert werden kann, um die entsprechende 13β-Alkyl-23-(O-alkyloxim)-LL-F28249-Verbindung der zuvor gezeigten Formel (I) zu liefern.

**4.** Verfahren nach Anspruch 3, in dem in der erhaltenen Verbindung
$R_1$ Methyl, Ethyl oder Isopropyl ist;
$R_2$ Methyl ist;
$R_3$ Wasserstoff, ($C_1$-$C_4$)-Alkyl oder

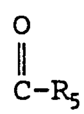

ist;
$R_5$ Wasserstoff, ($C_1$-$C_4$)-Alkoxymethyl, ($C_1$-$C_4$)-Halogenmethyl oder Formyl ist;
$R_4$ Isopropyl ist;
X $NOR_6$ ist; und
$R_6$ ($C_1$-$C_4$)-Alkyl ist.

**5.** Verfahren zum Schützen von Feldfrüchten, Bäumen, Sträuchern, gelagertem Korn und Zierpflanzen vor dem Angriff durch ein Insekt, eine Milbe und einen Fadenwurm, dadurch gekennzeichnet, daß man auf die Feldfrüchte, Bäume, Sträucher, das gelagerte Korn und die Zierpflanzen eine insektizid, akarizid oder nematizid wirksame Menge einer nach dem Verfahren von Anspruch 1 erhältlichen Verbindung anwendet.

**6.** Verfahren nach Anspruch 5, in dem die Verbindung die in Anspruch 1 dargestellte Strukturformel aufweist und
$R_1$ Isopropyl ist;
$R_7$ Wasserstoff ist und $R_8$ $OR_2$ ist;
$R_2$ Wasserstoff, ($C_1$-$C_4$)-Alkyl oder

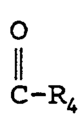

ist;
$R_4$ Wasserstoff, Methyl, Chlormethyl, Dichlormethyl, Trichlormethyl oder Methoxymethyl ist;
$R_3$ Methyl ist;
X Fluor ist;
W =N-Y ist;
Y $OR_5$ ist; und
$R_5$ ($C_1$-$C_4$)-Alkyl ist.

## Revendications

## Revendications pour les Etats contractants suivants : AT, BE, CH, DE, DK, FR, GB, IT, LI, LU, NL, SE

**1.** Composé répondant à la formule développée :

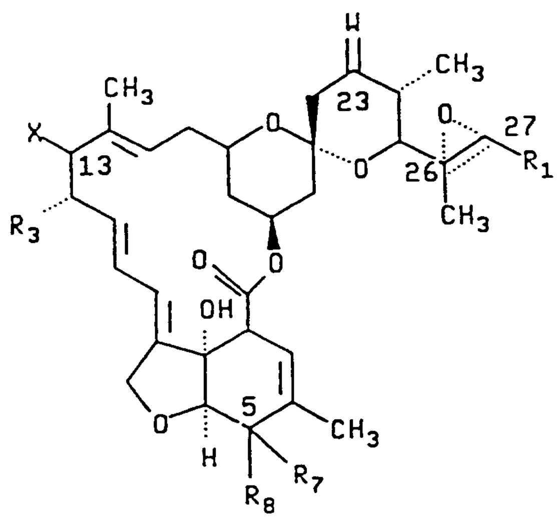

dans laquelle

- $R_1$ est un groupe méthyle, un groupe éthyle ou un groupe isopropyle ;
- $R_7$ est un atome d'hydrogène et $R_8$ est $OR_2$, ou, avec l'atome de carbone auquel ils sont fixés, $R_7$ et $R_8$ représentent C=O ;
- $R_2$ est un atome d'hydrogène, un groupe alkyle en

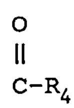 ;

$C_1$-$C_4$ ou
- $R_4$ est un atome d'hydrogène, un groupe alkyle en $C_1$-$C_4$, un groupe halogénoalkyle en $C_1$-$C_4$, un groupe alcoxy en $C_1$-$C_4$, un groupe phénoxy, un groupe (alcoxy en $C_1$-$C_4$)méthyle, un groupe phénoxyméthyle ou un groupe phényle facultativement substitué avec un groupe nitro, 1 à 3 atomes d'halogène, 1 à 3 groupes alkyle en $C_1$-$C_4$ ou 1 à 3 groupes alcoxy en $C_1$-$C_4$ ;
- $R_3$ est un atome d'hydrogène, un groupe méthyle ou un groupe éthyle ;
- X est un atome de fluor, de brome, de chlore ou d'iode ;
- W est =N-Y ;
- Y est $OR_5$ ou $NHR_6$ ;
- $R_5$ est un groupe alkyle en $C_1$-$C_4$ ou un groupe acyle en $C_1$-$C_4$ ;
- $R_6$ est un groupe acyle en $C_1$-$C_4$ ; et

la figure triangulaire en pointillé avec un atome d'oxygène en $C_{26}$-$C_{27}$ indique la présence soit d'une double liaison, soit d'un époxyde.

**2.** Composé selon la revendication 1, où

$R_1$ est un groupe isopropyle ;
$R_7$ est un atome d'hydrogène et $R_8$ est $OR_2$ ;
$R_2$ est un atome d'hydrogène, un groupe alkyle en $C_1$-$C_4$ ou

O
||
$C-R_4$ ;

$R_4$ est un atome d'hydrogène, un groupe méthyle, un groupe chlorométhyle, un groupe dichlorométhyle, un groupe trichlorométhyle ou un groupe méthoxyméthyle ;
$R_3$ est un groupe méthyle ;
X est un atome de fluor ;
W est = N - Y;
Y est $OR_5$ ; et
$R_5$ est un groupe alkyle en $C_1$-$C_4$.

**3.** Procédé de protection des cultures, des arbres, des arbustes, des céréales stockées et des plantes ornementales contre l'attaque par un insecte, un acarien et un nématode, caractérisé par l'application à ces cultures, arbres, arbustes, céréales stockées et plantes ornementales d'une quantité insecticide, acaricide ou nématicide efficace d'un composé répondant à la formule développée de la revendication 1.

**4.** Procédé selon la revendication 3, dans lequel le composé répond à la formule développée décrite dans la revendication 6 et

$R_1$ est un groupe isopropyle ;
$R_7$ est un atome d'hydrogène et $R_8$ est $OR_2$ ;
$R_2$ est un atome d'hydrogène, un groupe alkyle en $C_1$-$C_4$ ou

O
||
$C-R_4$ ;

$R_4$ est un atome d'hydrogène, un groupe méthyle, un groupe chlorométhyle, un groupe dichlorométhyle, un groupe trichlorométhyle ou un groupe méthoxyméthyle ;
$R_3$ est un groupe méthyle ;
X est un atome de fluor ;
W est =N-Y;
Y est $OR_5$ ; et
$R_5$ est un groupe alkyle en $C_1$-$C_4$.

**5.** Composition pour l'élimination des endo- et ectoparasites, des insectes, des acariens et des nématodes, caractérisée en ce qu'elle est constituée d'un support acceptable en agronomie contenant une quantité prophylactique, thérapeutique, pharmaceutique ou insecticide efficace d'un composé répondant à la formule développée de la revendication 1.

**6.** Composé répondant à la formule développée :

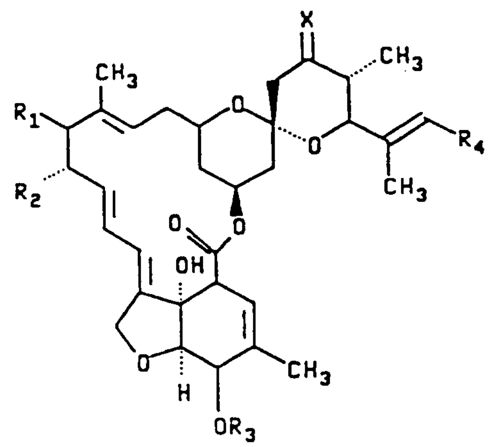

(I)

dans laquelle

- $R_1$ est un groupe alkyle en $C_1$-$C_4$ ;
- $R_2$ est un atome d'hydrogène, un groupe méthyle ou un groupe éthyle ;
- $R_3$ est un atome d'hydrogène, un groupe alkyle en $C_1$-$C_4$ ou

$$\overset{\displaystyle O}{\overset{\|}{C}}-R_5 \;;$$

- $R_5$ est un atome d'hydrogène, un groupe alkyle en $C_1$-$C_4$, un groupe halogénoalkyle en $C_1$-$C_4$, un groupe alcoxy en $C_1$-$C_4$, un groupe phénoxy, un groupe (alcoxy en $C_1$-$C_4$)méthyle, un groupe phénoxyméthyle ou un groupe phényle facultativement substitué avec un groupe nitro, 1 à 3 atomes d'halogène, 1 à 3 groupes alkyle en $C_1$-$C_4$ ou 1 à 3 groupes alcoxy en $C_1$-$C_4$ ;
- $R_4$ est un groupe méthyle, un groupe éthyle ou un groupe isopropyle ;
- X est un atome d'oxygène, $NOR_6$ ou $N$-$NHR_7$ ;
- $R_6$ est un atome d'hydrogène, un groupe alkyle en $C_1$-$C_4$, un groupe (alcoxy en $C_1$-$C_4$)méthyle, un groupe alcanoyle en $C_1$-$C_4$, un groupe benzyle ou un groupe phényle ; et
- $R_7$ est un groupe acyle en $C_1$-$C_4$, un groupe alkyle en $C_1$-$C_4$ ou un groupe benzoyle.

**7.** Composé selon la revendication 6, où

- $R_1$ est un groupe méthyle, un groupe éthyle ou un groupe isopropyle ;
- $R_2$ est un groupe méthyle ;
- $R_3$ est un atome d'hydrogène, un groupe alkyle en $C_1$-$C_4$ ou

$$\overset{\displaystyle O}{\overset{\|}{C}}-R_5 \;;$$

- $R_5$ est un atome d'hydrogène, un groupe (alcoxy en $C_1$-$C_4$)méthyle, un groupe halogénométhyle en $C_1$-$C_4$ ou un groupe formyle ;
- $R_4$ est un groupe isopropyle ;
- X est $NOR_6$ ; et
- $R_6$ est un groupe alkyle en $C_1$-$C_4$.

**8.** Composition pour l'élimination des endo- et ectoparasites, des insectes, des acariens et des nématodes, caractérisée en ce qu'elle est constituée d'un support acceptable en agronomie contenant une quantité prophylactique, thérapeutique, pharmaceutique ou insecticide efficace d'un composé de la revendication 6.

**Revendications pour les Etats contractants suivants : ES, GR**

**1.** Procédé pour la préparation des dérivés 13-halo-23-imino des composés LL-F 28249 de formule générale :

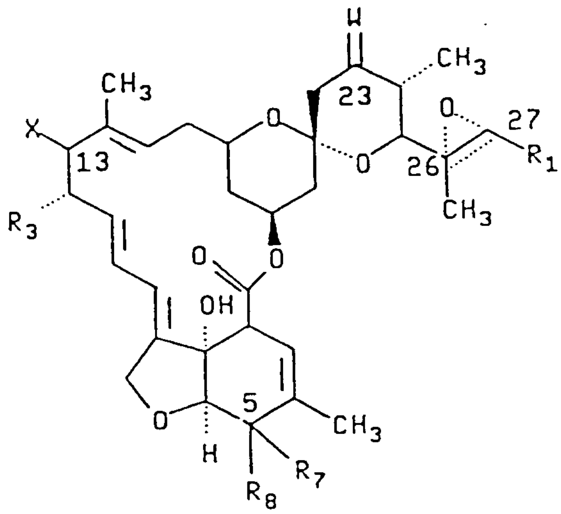

dans laquelle

$R_1$ est un groupe méthyle, un groupe éthyle ou un groupe isopropyle ;

$R_7$ est un atome d'hydrogène et $R_8$ est $OR_2$, ou, avec l'atome de carbone auquel ils sont fixés, $R_7$ et $R_8$ représentent C = O ;

$R_2$ est un atome d'hydrogène, un groupe alkyle en

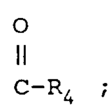

$C_1$-$C_4$ ou

$R_4$ est un atome d'hydrogène, un groupe alkyle en $C_1$-$C_4$, un groupe halogénoalkyle en $C_1$-$C_4$, un groupe alcoxy en $C_1$-$C_4$, un groupe phénoxy, un groupe (alcoxy en $C_1$-$C_4$)méthyle, un groupe phénoxyméthyle ou un groupe phényle facultativement substitué avec un groupe nitro, 1 à 3 atomes d'halogène, 1 à 3 groupes alkyle en $C_1$-$C_4$ ou 1 à 3 groupes alcoxy en $C_1$-$C_4$ ;

$R_3$ est un atome d'hydrogène, un groupe méthyle ou un groupe éthyle ;

X est un atome de fluor, de brome, de chlore ou d'iode ;

W est = N-Y ;

Y est $OR_5$ ou $NHR_6$ ;

$R_5$ est un groupe alkyle en $C_1$-$C_4$ ou un groupe acyle en $C_1$-$C_4$ ;

$R_6$ est un groupe acyle en $C_1$-$C_4$ ; et

la figure triangulaire en pointillé avec un atome d'oxygène en $C_{26}$-$C_{27}$ indique la présence soit d'une double liaison, soit d'un époxyde,

comprenant :

A) la réaction d'un composé 13-hydroxy-5,23-dioxo-LL-F 28249 avec un réactif d'halogénation de façon à obtenir le composé correspondant 13-halo-5,23-dioxo-LL-F 28249 que l'on fait réagir ensuite avec une alcoxylamine ou une hydroxylamine et du borohydrure de sodium de façon à obtenir le

composé correspondant 13-halo-23-imino-LL-F 28249; ou bien,

B) la réaction d'un composé 5-hydroxy-13-halo-23-imino-LL-F 28249 avec un agent alkylant ou acylant de façon à obtenir le composé correspondant 5-alkyloxy- ou 5-acyloxy-13-halo-23-imino-LL-F 28249; ou bien,

C) la réaction d'un composé 13-hydroxy-23-oxo-LL-F 28249 avec un réactif d'halogénation, suivie de la réaction avec une alcoxylamine ou avec une hydroxylamine de façon à obtenir le composé correspondant 13-halo-23-imino-5-acyloxy-LL-F 28249, qui peut-être, le cas échéant, hydrolysé en présence d'une base de façon à obtenir le composé correspondant 13-halo-23-imino-LL-F 28249; ou bien,

D) la réaction d'un composé 13-halo-23-oxo-LL -F 28249 avec l'acide méta-chloroperbenzoique de façon à obtenir le composé correspondant 13-halo-23-oxo-26,27-époxy-LL-F 28249 que l'on fait ensuite réagir avec une alcoxyle, acyloxyle ou hydroxyle oxime de façon à obtenir les dérivés correspondants 23-(O-alkyloxime)- ou 23-(O-acyloxime)- ou 23-oxime- des composés 13-halo-26,27-époxy-LL-F 28249.

**2.** Procédé selon la revendication 1 caractérisé en ce que le produit obtenu est tel que

$R_1$ est un groupe isopropyle ;
$R_7$ est un atome d'hydrogène et $R_8$ est $OR_2$ ;
$R_2$ est un atome d'hydrogène, un groupe alkyle en $C_1$-$C_4$ ou

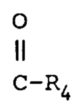 ;

$R_4$ est un atome d'hydrogène, un groupe méthyle, un groupe chlorométhyle, un groupe dichlorométhyle, un groupe trichlorométhyle ou un groupe méthoxyméthyle ;
$R_3$ est un groupe méthyle ;
X est un atome de fluor ;
W est = N - Y;
Y est $OR_5$ ; et
$R_5$ est un groupe alkyle en $C_1$-$C_4$.

**3.** Procédé pour la préparation des dérivés 13-alkyl-23-imino des composés LL-F 28249 de formule générale :

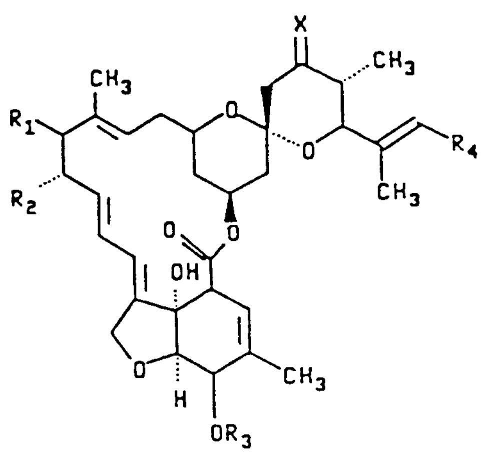

(I)

dans laquelle

$R_1$ est un groupe alkyle en $C_1$-$C_4$ ;
$R_2$ est un atome d'hydrogène un groupe méthyle ou un groupe éthyle ;

$R_3$ est un atome d'hydrogène, un groupe alkyle en $C_1$-$C_4$ ou

$$\overset{\overset{\displaystyle O}{\|}}{C}-R_5 \;;$$

$R_5$ est un atome d'hydrogène, un groupe alkyle en $C_1$-$C_4$, un groupe halogénoalkyle en $C_1$-$C_4$, un groupe alcoxy en $C_1$-$C_4$, un groupe phénoxy, un groupe (alcoxy en $C_1$-$C_4$)méthyle, un groupe phénoxyméthyle ou un groupe phényle facultativement substitué avec un groupe nitro, 1 à 3 atomes d'halogène, 1 à 3 groupes alkyle en $C_1$-$C_4$ ou 1 à 3 groupes alcoxy en $C_1$-$C_4$ ;

$R_4$ est un groupe méthyle, un groupe éthyle ou un groupe isopropyle ;

X est un atome d'oxygène, $NOR_6$ ou N-$NHR_7$ ;

$R_6$ est un atome d'hydrogène, un groupe alkyle en $C_1$-$C_4$, un groupe (alcoxy en $C_1$-$C_4$)méthyle, un groupe alcanoyle en $C_1$-$C_4$, un groupe benzyle ou un groupe phényle ; et

$R_7$ est un groupe acyle en $C_1$-$C_4$, un groupe alkyle en $C_1$-$C_4$ ou un groupe benzoyle.

comprenant la réaction d'un composé 5-acétoxy-13$\beta$-[éthoxycarbonyloxy]-2-{[(méthoxycarbonyl)-carbonyl]oxy}-LL-F 28249 avec un trialkylaluminium de façon à obtenir le composé correspondant 5-acétoxy-13$\beta$-alkyl-LL-F 28249 que l'on fait ensuite réagir avec le N-oxyde de la 4-méthylmorpholine et du pérruthénate de tétrapropylamonium de façon à obtenir le composé correspondant 5-acétoxy-13$\beta$-alkyl-23-oxo-LL-F 28249, suivie, le cas échéant du traitement par un chlorhydrate d'alcoxylamine de façon à obtenir le composé correspondant 5-acétoxy-13$\beta$-alkyl-23-(O-alkyloxime)-LL-F 28249, lequel peut-être, le cas échéant, hydrolysé en présence d'une base forte de façon à obtenir le composé correspondant 13$\beta$ -alkyl-23-(O-alkyloxime)-RL-F 28249 de formule (I) schématisé ci-dessus.

**4.** Procédé selon la revendication 3, caractérisé en ce que le composé obtenu est tel que :

$R_1$ est un groupe méthyle, un groupe éthyle ou un groupe isopropyle ;

$R_2$ est un groupe méthyle ;

$R_3$ est un atome d'hydrogène, un groupe alkyle en $C_1$-$C_4$ ou

$$\overset{\overset{\displaystyle O}{\|}}{C}-R_5 \;;$$

$R_5$ est un atome d'hydrogène, un groupe (alcoxy en $C_1$-$C_4$)méthyle, un groupe halogénométhyle en $C_1$-$C_4$ ou un groupe formyle ;

$R_4$ est un groupe isopropyle ;

X est $NOR_6$ ; et

$R_6$ est un groupe alkyle en $C_1$-$C_4$

**5.** Procédé de protection des cultures, des arbres, des arbustes, des céréales stockées et des plantes ornementales contre l'attaque par un insecte, un acarien et un nématode, caractérisé par l'application à ces cultures, arbres, arbustes, céréales stockées et plantes ornementales d'une quantité insecticide, acaricide ou nématicide efficace d'un composé répondant à la formule développée de la revendication 1.

**6.** Procédé selon la revendication 5, dans lequel le composé répond à la formule développée décrite dans la revendication 1, dans laquelle

$R_1$ est un groupe isopropyle ;

$R_7$ est un atome d'hydrogène et $R_8$ est $OR_2$ ;

$R_2$ est un atome d'hydrogène, un groupe alkyle en $C_1$-$C_4$ ou

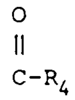 ;

- $R_4$ est un atome d'hydrogène, un groupe méthyle, un groupe chlorométhyle, un groupe dichlorométhyle, un groupe trichlorométhyle ou un groupe méthoxyméthyle ;
- $R_3$ est un groupe méthyle ;
- X est un atome de fluor ;
- W est = N - Y;
- Y est $OR_5$ : et
- $R_5$ est un groupe alkyle en $C_1$-$C_4$.